# EUROPEAN PATENT SPECIFICATION

(11) **EP 3 996 657 B1**
(45) Date of publication and mention of the grant of the patent: **23.10.2024**
(21) Application number: 20735601.5
(22) Date of filing: 03.07.2020
(51) Int. Cl.: A61K 8/02, A61K 8/19, A61Q 19/00

(54) **DRY COSMETIC AND/OR SKIN CARE COMPOSITION**
TROCKENE KOSMETIK- UND/ODER HAUTPFLEGEZUSAMMENSETZUNG
COMPOSITION COSMÉTIQUE SÈCHE ET/OU DE SOINS CUTANÉS

(30) Priority: 08.07.2019 EP 19185003
(43) Date of publication of application: 18.05.2022
(73) Proprietor: Omya International AG, 4665 Oftringen (CH)
(72) Inventor: SHARMA, Lalit, 4800 Zofingen (CH); BUDDE, Tanja, 4805 Brittnau (CH); LANDER, Stefan, 5102 Rupperswil (CH)
(74) Representative: Maiwald GmbH
(86) International application number: PCT/EP2020/068838
(87) International publication number: WO 2021/004942

(56) References cited:
- EP-A1- 3 360 601
- EP-A1- 3 385 335
- WO-A1-2016/102134
- US-A1- 2015 218 381
- US-A1- 2016 271 025
- US-A1- 2018 051 175

## Description

The present invention refers to a dry cosmetic and/or skin care composition, a process for producing the dry cosmetic and/or skin care composition, the use of the dry composition as a cosmetic and/or skin care composition, and the use of a mixture comprising a first component being a natural ground calcium carbonate, and a second component being a surface-reacted calcium carbonate as a replacement for talc or a talc-containing material in a cosmetic and/or skin care composition.

Dry cosmetic and/or skin care compositions are used for a variety of different applications. For example, a dry cosmetic and/or skin care compositions may be applied for modifying the skin appearance by absorbing moisture and/ or body fluids such as sebum, lipids, sweat, or urine. The absorption of moisture, sebum, lipids, sweat or other body fluids from the skin surface may provide the skin with a matt look, which is often associated with a cleaner and/or a more natural skin appearance. Dry cosmetic and/or skin care compositions may also be used to color the skin, to hide blemishes, conceal or diminish fine lines or wrinkles, minimize pores or to change and/or even out the skin tone.

Furthermore, dry cosmetic and/or skin care compositions are used to modify the skin feel. For example, the absorption of water, residual moisture and/or body fluids such as sebum, lipids or sweat from the skin surface can provide a smoothened skin having a softer skin feel and/or causing less friction with e.g. clothing, diapers, gloves and/or mechanic or electronic shavers. In addition thereto, the absorption of body fluids may also provide the user with a dry and/or comfortable skin feeling during and/or after application of the dry composition, and/or may avoid the development of unpleasant odors.

Besides providing one or more of the aforementioned effects, a dry cosmetic and/or skin care composition usually has to fulfil further criteria to be fully accepted by the customer. For example, the dry cosmetic and/or skin care composition has to adhere properly to the skin, must be easily applicable by the user and/or should provide the user with a pleasant feeling during application.

Furthermore, the dry cosmetic and/or skin care composition should be free of any components, which may be considered a health concern for the user. For example, certain silicates such as talc or talc-containing material are believed to increase the risk of lung conditions, if accidentally inhaled due to wrong use of the composition and/or use thereof in excess. In view thereof, customers usually prefer dry cosmetic and/or skin care composition, which are free of components such as talc or talc-containing material. By using a dry cosmetic and/or skin care composition being free of e.g. talc or talc-containing material, a potential health risk through unintentional inhalation of the composition might be minimized or avoided. Usually, unintentional inhalation of the dry cosmetic and/or skin care composition is of special concern, if dry cosmetic and/or skin care compositions are applied to the skin of a baby in form of a baby powder. Another health concern relates to the use of dry cosmetic and/or skin care composition containing talc or talc-containing material in sensitive body areas such as the genital area. For example, lawsuits in the United States of America alleged that the use of talcum powder in the female genital area may increase the risk of ovarian cancer.

EP3360601 A1 relates to a cosmetic composition having UV-A and/or UV-B protection comprising at least one inorganic UV filter, and surface-reacted calcium carbonate having a volume median particle size *d* ₅₀ from 0.1 to 90 µm, wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donors treatment and/or is supplied from an external source. WO2016/102134 A1 discloses a powdery cosmetic composition comprising: (i) at least 50 wt% of a cosmetically acceptable powdery base; (ii) 2 wt% to 40 wt% precipitated calcium carbonate; and, (iii) 0.0001 wt% to 5 wt% fragrance ingredient wherein said precipitated calcium carbonate is uncoated and of calcite form. US2016/271025 A1 relates to an abrasive cleaning composition comprising at least 6 wt.-%, based on the total weight of the composition, of a surface-reacted calcium carbonate as an abrasive material, wherein the surface-reacted calcium carbonate is a reaction product of natural or synthetic calcium carbonate with carbon dioxide and at least one acid. US 2015/218381 A1 describes a surface treated calcium carbonate and method for making the same. The calcium carbonate is surface treated with a hydrophobic compound, such as a trialkoxyalkylsilane, fluoro high purity ammonium C6-16 perfluoroalkyl phosphate, or dimethicone trimethylsiloxysilane. A personal care product comprising the surface treated calcium carbonate is also described in US 2015/218381 A1. EP3385335 A1 relates to an aqueous pigment composition for use in paints or coatings. The aqueous pigment composition comprises a blend of ground natural calcium carbonate (GNCC) and surface modified calcium carbonate (MCC) at a specific ratio and each having a specific particle size distribution. US2018/051175 A1 relates to a process for producing an aqueous slurry comprising a blend of surface modified calcium carbonate comprising particles (MCC) and precipitated calcium carbonate comprising particles (PCC), an aqueous slurry comprising the blend, as well as the blend obtainable by drying the aqueous slurry and their uses in paper, paper coating, tissue paper, digital photo paper, paints, coatings, adhesives, plastics, waste water treating or waste water treating agents.

There is a continuing need in the art for dry cosmetic and/or skin care compositions, which show an alternative, or an improved, skin appearance modification and/or skin feel modification. There is also a need in the art for a dry cosmetic and/or skin care composition, which more effectively decreases the friction of the skin with an object being in contact therewith than known products. There is also a need in the art for a dry cosmetic and/or skin care composition, which is better or easier applicable to the skin than known products. Furthermore, there is a need in the art for a dry cosmetic and/or skin care composition, which is free of components being considered a health concern such as talc or talc-containing material. In particular, there is a demand for a cosmetic and/or skin care composition, especially a baby powder and/or a body powder, which is free of talc or talc-containing material.

Accordingly, an objective of the present invention may be seen in the provision of a dry cosmetic and/or skin care composition, which shows an alternative, or an improved, skin appearance modification and/or skin feel modification. Another objective of the present invention may be seen in the provision of a dry cosmetic and/or skin care composition, which more effectively decreases the friction of the skin with an object being in contact therewith. Yet another objective of the present invention may be seen in the provision of a dry cosmetic and/or skin care composition, which is better or easier applicable to the skin. Still another objective of the present invention may be seen in the provision of a dry cosmetic and/or skin care composition, which is free of components being considered a health concern such as talc or talc-containing material. Yet another objective of the present invention may be seen in the provision of a dry cosmetic and/or skin care composition, especially a baby powder and/or a body powder, which is free of talc or talc-containing material. Still another objective of the present invention may be seen in the provision of a dry cosmetic and/or skin care composition, especially a baby powder and/or a body powder, which is free of talc or talc-containing material and provides at least satisfactory modification of skin appearance, skin feel, skin smoothness and satisfactory applicability.

One or more of the foregoing objections is/are solved by the present invention.

According to one aspect of the present invention, a dry cosmetic and/or skin care composition is provided. The dry cosmetic and/or skin care composition comprises a mixture of a first component being a natural ground calcium carbonate, and a second component being a surface-reacted calcium carbonate, wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source. The dry cosmetic and/or skin care composition contains a water content of less than 15 wt.%, based on the total weight of the cosmetic and/or skin care composition

The inventors surprisingly found that a dry cosmetic and/or skin care composition according to the invention, e.g. in form of a loose baby powder or body powder, is well applicable to the skin. For example, it has a good skin adherence, is well spreadable and has little resistance during application onto the skin. The application of the dry cosmetic and/or skin care composition according to the invention leaves the skin with a soft and dry touch. Furthermore, the dry cosmetic and/or skin care composition according to the invention does not require components such as talc or talc-containing materials, which are considered a health concern by certain customers.

According to another aspect of the present invention, a process for preparing a dry cosmetic and/or skin care composition is provided. The process comprises the following steps: a) providing a first component being a natural ground calcium carbonate, b) providing a second component being a surface-reacted calcium carbonate, wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, c) mixing the first component of step a) with the second component of step b).

According to another aspect of the present invention, the use of a dry composition as a cosmetic and/or skin care composition, preferably as a cosmetic and/or skin care powder, and most preferably as a baby powder and/or a body powder, is provided. The dry composition comprises a mixture of a first component being a natural ground calcium carbonate, and a second component being a surface-reacted calcium carbonate, wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source.

The inventors surprisingly found that a dry composition as described herein is useful as a dry cosmetic and/or skin care composition in that it effectively provides moisture and/or body fluid absorption, skin feel modification and/or skin appearance modification.

According to another aspect of the present invention, the use of a mixture as a replacement for talc or a talc-containing material in a dry cosmetic and/or skin care composition is provided. The mixture comprises a first component being a natural ground calcium carbonate, and a second component being a surface-reacted calcium carbonate, wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source.

The inventors surprisingly found that the mixture of the first component and a second component as described herein mimics to a certain extent the characteristics of talc as a component of a dry cosmetic and/or skin care composition such as a baby and/or body powder. In particular, it has been found that the use of a mixture of the first component and a second component as described herein in a dry cosmetic and/or skin care composition has similar, or even improved, properties as regards skin feel during and/or after application, skin appearance, moisture and body fluid absorption compared to a dry cosmetic and/or skin care composition, which contains a comparable or the same amount of talc to provide these properties.

Advantageous embodiments of the dry cosmetic and/or skin care composition, the process for producing such a composition, and the uses thereof are defined in the corresponding sub-claims.

It is to be understood that all embodiments described herein of the dry cosmetic and/or skin care composition according to the present invention are also embodiments of the process for producing the inventive composition and for the uses thereof. Likewise, all embodiments described herein of the first component and/or the second component being present in the composition according to the present invention are also embodiments of the process for producing the inventive composition and for the uses thereof.

According to one embodiment of the present invention, the dry cosmetic and/or skin care composition is a powder, preferably a baby powder and/or a body powder.

According to one embodiment of the present invention, the first component is present in an amount of from 1 wt.% to 99 wt.%, preferably from 30 wt.% to 99 wt.%, more preferably from 50 wt.% to 95 wt.%, even more preferably from 60 wt.% to 95 wt.%, and most preferably from 70 wt.% to 90 wt.%, based on the total weight of the first component and the second component, and the second component is present in an amount of from 1 wt.% to 99 wt.%, preferably from 1 wt.% to 70 wt.%, more preferably from 5 wt.% to 50 wt.%, even more preferably from 5 wt.% to 40 wt.%, and most preferably from 10 wt.% to 30 wt.%, based on the total weight of the first component and the second component.

According to one embodiment of the present invention, the first component is a natural ground calcium carbonate selected from the group consisting of marble, chalk, limestone, and mixtures thereof.

According to one embodiment of the present invention, the surface-reacted calcium carbonate is a reaction product of natural ground calcium carbonate selected from the group consisting of marble, chalk, limestone, and mixtures thereof, with carbon dioxide and at least one H₃O⁺ ion donor, wherein the carbon dioxide is formed in situ by the at least one H₃O⁺ ion donor treatment and/or is supplied from an external source, or the surface-reacted calcium carbonate is a reaction product of precipitated calcium carbonate selected from the group consisting of precipitated calcium carbonates having an aragonitic, vateritic or calcitic crystal form, and mixtures thereof, with carbon dioxide and at least one H₃O⁺ ion donor, wherein the carbon dioxide is formed in situ by the at least one H₃O⁺ ion donor treatment and/or is supplied from an external source.

According to one embodiment of the present invention, the at least one H₃O⁺ ion donor is selected from the group consisting of hydrochloric acid, sulphuric acid, sulphurous acid, phosphoric acid, citric acid, oxalic acid, an acidic salt, acetic acid, formic acid, and mixtures thereof, preferably the at least one H₃O⁺ ion donor is selected from the group consisting of hydrochloric acid, sulphuric acid, sulphurous acid, phosphoric acid, oxalic acid, H₂PO₄⁻, being at least partially neutralised by a cation selected from Li⁺, Na⁺ and/or K⁺, HPO₄²⁻, being at least partially neutralised by a cation selected from Li⁺, Na^{+,} K⁺, Mg²⁺, and/or Ca²⁺, and mixtures thereof, more preferably the at least one H₃O⁺ ion donor is selected from the group consisting of hydrochloric acid, sulphuric acid, sulphurous acid, phosphoric acid, oxalic acid, or mixtures thereof, and most preferably, the at least one H₃O⁺ ion donor is phosphoric acid.

According to one embodiment of the present invention, the first component has a volume median particle size *d*₅₀ from 0.1 to 50 µm, preferably from 0.5 to 40 µm, more preferably from 0.5 to 20 µm, even more preferably from 0.5 to 10 µm, and most preferably from 0.8 to 8 µm, and/or the first component has a specific surface area of from 0.5 m²/g to 30 m²/g, preferably from 1 m²/g to 20 m²/g, and more preferably from 2 m²/g to 15 m²/g, measured using nitrogen and the BET method.

According to one embodiment of the present invention, the second component has a volume median particle size *d*₅₀ from 0.5 to 50 µm, preferably from 1 to 40 µm, more preferably from 1.2 to 30 µm, and even more preferably from 1.5 to 15 µm, and most preferably from 3 to 10 µm, and/or the second component has a specific surface area of from 15 m²/g to 200 m²/g, preferably from 20 m²/g to 180 m²/g, and preferably from 25 m²/g to 160 m²/g, and most preferably from 30 m²/g to 90 m²/g measured using nitrogen and the BET method.

According to one embodiment of the present invention, the dry cosmetic and/or skin care composition is free of talc or a talc-containing material.

According to one embodiment of the present invention, the dry cosmetic and/or skin care composition comprises one or more of a further component, preferably the one or more further component is selected from the group consisting of a fragrance, an aroma, an antibacterial and/or an antiseptic agent, a fatty acid or a salt thereof, a fatty alcohol, a vegetable or a synthetic oil, a polymeric carbohydrate, a mineral additive, a pigment, a salt, and mixtures thereof.

According to one embodiment of the present invention, the first component of step a) and the second component of step b) is provided in dry form and mixing step c) is a dry blending step, or the first component of step a) and/or the second component of step b) is provided in form of an aqueous suspension, preferably in form of a slurry, and the process further comprises a step d) of drying the mixture obtained in step c), preferably step d) is a spray drying step or a superheated steam drying step, more preferably a spray drying step.

According to one embodiment of the present invention, the composition is used for absorbing fluids, for decreasing skin friction, for modifying the skin feel, and/or for modifying the skin appearance.

It should be understood that for the purposes of the present invention, the following terms have the following meanings:
A dry "cosmetic and/or skin care" composition in the meaning of the present invention refers to a dry composition that is applied onto the skin and that does not contain harmful and/or irritating substances, and/or does not contain substances, which are not approved for use in a cosmetic and/or skin care composition. Substances, which are not approved for use in cosmetics may, for example, be found in "REGULATION (EC) No 1223/2009 OF THE EUROPEAN PARLIAMENT AND OF THE COUNCIL". A dry "cosmetic and/or skin care" composition does not encompass a composition that is typically taken up orally or is typically used as a personal care product for other parts of the body such as teeth, hair, fingernails etc.. A "dry" cosmetic and/or skin care composition in the meaning of the present invention refers to a cosmetic and/or skin care composition, which contains a water content of less than 15 wt.%, preferably less than 10.0 wt.%, and more preferably less than 5.0 wt.%, based on the total weight of the cosmetic and/or skin care composition. A suitable method for determining the water content of the dry cosmetic and/or skin care composition will be selected by the skilled person. For example, the water content may be determined according to the Coulometric Karl Fischer measurement method, wherein the composition is heated to 220 °C, and the water content released as vapour and isolated using a stream of nitrogen gas (at 100 ml/min) is determined in a Coulometric Karl Fischer unit.

"Natural ground calcium carbonate" (GCC) in the meaning of the present invention is a calcium carbonate obtained from natural sources, such as limestone, marble, or chalk, and processed through a wet and/or dry treatment such as grinding, screening and/or fractionating, for example, by a cyclone or classifier.

"Precipitated calcium carbonate" (PCC) in the meaning of the present invention is a synthesised material, obtained by precipitation following reaction of carbon dioxide and lime in an aqueous, semi-dry or humid environment or by precipitation of a calcium and carbonate ion source in water. PCC may be in the vateritic, calcitic or aragonitic crystal form. PCCs are described, for example, in EP2447213 A1, EP2524898 A1, EP2371766 A1, EP1712597 A1, EP1712523A1, or WO2013142473 A1.

The term "surface-reacted" in the meaning of the present application shall be used to indicate that a material has been subjected to a process comprising partial dissolution of said material upon treatment with an H₃O⁺ ion donor (e.g., by use of water-soluble free acids and/or acidic salts) in aqueous environment followed by a crystallization process which may occur in the absence or presence of further crystallization additives.

An "H₃O⁺ ion donor" in the context of the present invention is a Brønsted acid and/or an acid salt, i.e. a salt containing an acidic hydrogen.

The term "acid" as used herein refers to an acid in the meaning of the definition by Brønsted and Lowry (e.g., H₂SO₄, HSO₄⁻).

The term "free acid" refers only to those acids being in the fully protonated form (e.g., H₂SO₄).

A cosmetic and/or skin care "powder" in the meaning of the present invention refers to a cosmetic and/or skin care composition, which only contains particulate components having a weight median particle size *d*₅₀ of not more than 200 µm, preferably of not more than 100 µm, more preferably of not more than 80 µm, and most preferably of not more than 60 µm.

The "particle size" of particulate materials is described by its distribution of particle sizes *dₓ*. Unless indicated otherwise, the value *dₓ* represents the diameter relative to which *x* % by weight of the particles have diameters less than *dₓ*. This means that, for example, the *d*₂₀ value is the particle size at which 20 wt.-% of all particles are smaller than that particle size. The *d*₅₀ value is thus the weight median particle size, i.e. 50 wt.-% of all particles are smaller than this particle size. For the purpose of the present invention, the particle size is specified as weight median particle size *d*₅₀(wt.) unless indicated otherwise. Particle sizes were determined by using a Sedigraph^{™} 5100 instrument or Sedigraph^{™} 5120 instrument of Micromeritics Instrument Corporation. The method and the instrument are known to the skilled person and are commonly used to determine the particle size of fillers and pigments. The measurements were carried out in an aqueous solution of 0.1 wt.-% Na₄P₂O₇.

For certain materials specified herein, the "particle size" is described as volume-based particle size distribution. This is indicated, for example, by "volume based median particle size", "volume median particle size" or "volume top cut particle size". Volume median particle size *d*₅₀ was evaluated using a Malvern Mastersizer 2000 or 3000 Laser Diffraction System. The *d*₅₀ or *d*₉₈ value, measured using a Malvern Mastersizer 2000 or 3000 Laser Diffraction System, preferably a Malvern Mastersizer 3000 Laser Diffraction System, indicates a diameter value such that 50 % or 98 % by volume, respectively, of the particles have a diameter of less than this value. The raw data obtained by the measurement are analysed using the Mie theory, with a particle refractive index of 1.57 and an absorption index of 0.005. The measurements were carried out in an aqueous solution of 0.1 wt.-% Na₄P₂O₇.

The term "particulate" in the meaning of the present application refers to materials composed of a plurality of particles. Said plurality of particles may be defined, for example, by its particle size distribution. The expression "particulate material" may comprise granules, powders, grains, tablets, or crumbles.

The "specific surface area" (expressed in m²/g) of a material as used throughout the present document can be determined by the Brunauer Emmett Teller (BET) method with nitrogen as adsorbing gas and by use of a ASAP 2460 instrument from Micromeritics. The method is well known to the skilled person and defined in ISO 9277:2010. Prior to such measurements, the sample was filtered within a Büchner funnel, rinsed with deionised water and dried at 110°C in an oven for at least 12 hours. The total surface area (in m²) of said material can be obtained by multiplication of the specific surface area (in m²/g) and the mass (in g) of the material.

In the context of the present invention, the term "pore" is to be understood as describing the space that is found between and/or within particles, i.e. that is formed by the particles as they pack together under nearest neighbour contact (interparticle pores), such as in a powder or a compact and/or the void space within porous particles (intraparticle pores), and that allows the passage of liquids under pressure when saturated by the liquid and/or supports absorption of surface wetting liquids.

Unless specified otherwise, the term "drying" refers to a process according to which at least a portion of water is removed from a material to be dried such that a constant weight of the obtained "dried" material at 200°C is reached. Moreover, a "dried" or "dry" material may be defined by its total moisture content which, unless specified otherwise, is less than or equal to 1.0 wt.-%, preferably less than or equal to 0.5 wt.-%, more preferably less than or equal to 0.2 wt.-%, and most preferably between 0.03 and 0.07 wt.-%, based on the total weight of the dried material.

For the purpose of the present application, "water-insoluble" materials are defined as those which, when mixed with 100 ml of deionised water and filtered at 20°C to recover the liquid filtrate, provide less than or equal to 0.1 g of recovered solid material following evaporation at 95 to 100°C of 100 g of said liquid filtrate. "Water-soluble" materials are defined as materials leading to the recovery of greater than 0.1 g of solid material following evaporation at 95 to 100°C of 100 g of said liquid filtrate. In order to assess whether a material is an insoluble or soluble material in the meaning of the present invention, the sample size is greater than 0.1 g, preferably 0.5 g or more.

A "suspension" or "slurry" in the meaning of the present invention comprises undissolved solids and water, and optionally further additives, and usually contains large amounts of solids and, thus, is more viscous and can be of higher density than the liquid from which it is formed.

The expression "skin feel" in the meaning of the present invention refers to the feeling of the skin during and/or after the application of the dry cosmetic and/or skin care composition onto the skin surface. For example, the skin feel may relate to a soft, smooth, greasy, dry, tight and/or flexible feeling of the skin.

The expression "skin appearance" in the meaning of the present invention relates to the optical impression of the skin to the eye of the beholder during and/or after application of the dry cosmetic and/or skin care composition. For example, the skin may appear shiny, matt, evenly or unevenly toned.

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

In the following preferred embodiments of the inventive composition will be set out in more detail.

### The first component

The dry cosmetic and/or skin care composition according to the invention comprises a first component being a natural ground calcium carbonate.

The first component of the inventive dry cosmetic and/or skin care composition is present in dry form. For example, the first component may have a residual moisture content of below 3.0 wt.%, preferably of below 2.0 wt.%, and more preferably of below 1.0 wt.%, based on the total dry weight of the first component. The residual moisture content may be determined as described above for the dry cosmetic and/or skin care composition.

According to one embodiment, the first component has a volume median particle size *d*₅₀ from 0.1 to 50 µm, preferably from 0.5 to 40 µm, more preferably from 0.5 to 20 µm, even more preferably from 0.5 to 10 µm, and most preferably from 0.8 to 8 µm, and/or the first component has a specific surface area of from 0.5 m²/g to 30 m²/g, preferably from 1 m²/g to 20 m²/g, and more preferably from 2 m²/g to 15 m²/g, measured using nitrogen and the BET method. According to another embodiment, the first component has a volume median particle size *d*₅₀ from 0.1 to 50 µm, preferably from 0.5 to 40 µm, more preferably from 0.5 to 20 µm, even more preferably from 0.5 to 10 µm, and most preferably from 0.8 to 8 µm, and the first component has a specific surface area of from 0.5 m²/g to 30 m²/g, preferably from 1 m²/g to 20 m²/g, and more preferably from 2 m²/g to 15 m²/g, measured using nitrogen and the BET method.

The dry cosmetic and/or skin care composition according to the invention comprises a first component being a natural ground calcium carbonate.

It is appreciated that the natural ground calcium carbonate can be one specific natural ground calcium carbonate or a mixture of different kinds of natural ground calcium carbonate(s).

In one embodiment of the present invention, the natural ground calcium carbonate comprises, preferably consists of, one kind of natural ground calcium carbonate. Alternatively, the natural ground calcium carbonate comprises, preferably consists of, two or more kinds of natural ground calcium carbonates. For example, the natural ground calcium carbonate comprises, preferably consists of, two or three kinds of natural ground calcium carbonates. Preferably, the natural ground calcium carbonate comprises, more preferably consists of, one kind of natural ground calcium carbonate.

In one embodiment of the present invention, the natural ground calcium carbonate is a ground calcium carbonate-containing mineral, preferably the calcium carbonate-containing mineral is selected from the group consisting of chalk, limestone, marble, dolomite and mixtures thereof.

In a preferred embodiment of the present invention, the natural ground calcium carbonate is selected from the group consisting of chalk, limestone or marble. More preferably, the natural ground calcium carbonate is limestone or marble, and most preferably marble.

A natural ground calcium carbonate may be obtained, for example, in a wet and/or dry comminution step, such as crushing and/or grinding, from natural calcium carbonate-containing minerals (e.g. chalk, limestone, marble or dolomite). According to one embodiment, the natural ground calcium carbonate is a wet-natural ground calcium carbonate. In another embodiment, the natural ground calcium carbonate is a dry-natural ground calcium carbonate.

The grinding step can be carried out with any conventional grinding device, for example, under conditions such that refinement predominantly results from impacts with a secondary body, i.e. in one or more of a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. The grinding step may also be performed under conditions such that autogenous grinding takes place and/or by horizontal ball milling, and/or other such processes known to the skilled man.

In one embodiment, grinding is carried out in a vertical or horizontal ball mill, preferably in a vertical ball mill. Such vertical and horizontal ball mills usually consist of a vertically or horizontally arranged, cylindrical grinding chamber comprising an axially fast rotating agitator shaft being equipped with a plurality of paddles and/or stirring discs, such as described for example in EP0607840 A1.

It is to be noted that grinding of the calcium carbonate-containing mineral may be carried out by using at least one of the aforementioned grinding methods or devices. However, it is also possible to use a combination of any of the foregoing methods or a series of any of the aforementioned grinding devices.

Subsequent to the grinding step, the ground calcium carbonate-containing mineral may, optionally, be divided into two or more fractions, each having different particle distributions, by use of a classifying step. A classifying step in general serves to divide a feed fraction having a certain particle size distribution into a coarse fraction, which may be subjected to another grinding cycle, and a fine fraction, which may be used as the final product. For this purpose, screening devices as well as gravity-based devices, such as centrifuges or cyclones (e.g. hydrocyclones) and any combination of the aforementioned devices may be used.

The natural ground calcium carbonate may have specific physical characteristics such as a specific particle size and/or a specific surface area.

According to one embodiment, the natural ground calcium carbonate has a volume median particle size *d*₅₀ from 0.1 to 50 µm, preferably from 0.5 to 40 µm, more preferably from 0.5 to 20 µm, even more preferably from 0.5 to 10 µm, and most preferably from 0.8 to 8 µm, and/or a volume top cut particle size *d*₉₈ of from 2 to 80 µm, preferably from 2 to 60 µm, more preferably 2 to 40 µm, even more preferably from 3 to 30 µm, and most preferably from 4 to 20 µm.

According to one embodiment, the natural ground calcium carbonate has a specific surface area of from 0.5 m²/g to 30 m²/g, preferably from 1 m²/g to 20 m²/g, and more preferably from 2 m²/g to 15 m²/g, measured using nitrogen and the BET method.

According to one preferred embodiment, the first component is a natural ground calcium carbonate having a volume median particle size *d*₅₀ from 0.1 to 50 µm, preferably from 0.5 to 40 µm, more preferably from 0.5 to 20 µm, even more preferably from 0.5 to 10 µm, and most preferably from 0.8 to 8 µm, and a volume top cut particle size *d*₉₈ of from 2 to 80 µm, preferably from 2 to 60 µm, more preferably 2 to 40 µm, even more preferably from 3 to 30 µm, and most preferably from 4 to 20 µm, and a specific surface area of from 0.5 m²/g to 30 m²/g, preferably from 1 m²/g to 20 m²/g, and more preferably from 2 m²/g to 15 m²/g, measured using nitrogen and the BET method.

In one embodiment, the natural ground calcium carbonate has a volume median particle size *d*₅₀ from 0.8 to 8 µm, and a volume top cut particle size *d*₉₈ of from 4 to 20 µm, and a specific surface area of from 2 m²/g to 15 m²/g, measured using nitrogen and the BET method.

### The second component

The dry cosmetic and/or skin care composition according to the invention comprises a second component being a surface-reacted calcium carbonate. A surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source.

The second component of the inventive dry cosmetic and/or skin care composition is present in dry form. For example, the second component may have a residual moisture content of below 10 wt.%, preferably of below 5.0 wt.%, based on the total dry weight of the second component. For example, the residual moisture may be around 3.0 wt.%, based on the total dry weight of the second component. The residual moisture content may be determined as described above for the dry cosmetic and/or skin care composition.

It is appreciated that the surface-reacted calcium carbonate can be one or a mixture of different kinds of surface-reacted calcium carbonate(s).

In one embodiment of the present invention, the surface-reacted calcium carbonate comprises, preferably consists of, one kind of surface-reacted calcium carbonate. Alternatively, the surface-reacted calcium carbonate comprises, preferably consists of, two or more kinds of surface-reacted calcium carbonates. For example, the surface-reacted calcium carbonate comprises, preferably consists of, two or three kinds of surface-reacted calcium carbonates. Preferably, the surface-reacted calcium carbonate comprises, more preferably consists of, one kind of surface-reacted calcium carbonate.

In a preferred embodiment of the invention the surface-reacted calcium carbonate is obtained by a process comprising the steps of: (a) providing a suspension of natural or precipitated calcium carbonate, (b) adding at least one acid having a pKₐ value of 0 or less at 20°C or having a pKₐ value from 0 to 2.5 at 20°C to the suspension of step a), and (c) treating the suspension of step (a) with carbon dioxide before, during or after step (b). According to another embodiment the surface-reacted calcium carbonate is obtained by a process comprising the steps of: (A) providing a natural or precipitated calcium carbonate, (B) providing at least one water-soluble acid, (C) providing gaseous CO₂, (D) contacting said natural or precipitated calcium carbonate of step (A) with the at least one acid of step (B) and with the CO₂ of step (C), characterised in that: (i) the at least one acid of step B) has a pKₐ of greater than 2.5 and less than or equal to 7 at 20°C, associated with the ionisation of its first available hydrogen, and a corresponding anion is formed on loss of this first available hydrogen capable of forming a water-soluble calcium salt, and (ii) following contacting the at least one acid with natural or precipitated calcium carbonate, at least one water-soluble salt, which in the case of a hydrogen-containing salt has a pKₐ of greater than 7 at 20°C, associated with the ionisation of the first available hydrogen, and the salt anion of which is capable of forming water-insoluble calcium salts, is additionally provided.

"Natural ground calcium carbonate" (GCC) preferably is selected from calcium carbonate containing minerals selected from the group comprising marble, chalk, limestone and mixtures thereof. Natural ground calcium carbonate may comprise further naturally occurring components such as magnesium carbonate, alumino silicate etc.

In general, the grinding of natural ground calcium carbonate may be a dry or wet grinding step and may be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the calcium carbonate containing mineral material comprises a wet ground calcium carbonate containing mineral material, the grinding step may be performed under conditions such that autogenous grinding takes place and/or by horizontal ball milling, and/or other such processes known to the skilled man. The wet processed ground calcium carbonate containing mineral material thus obtained may be washed and dewatered by well-known processes, e.g. by flocculation, filtration or forced evaporation prior to drying. The subsequent step of drying (if necessary) may be carried out in a single step such as spray drying, or in at least two steps. It is also common that such a mineral material undergoes a beneficiation step (such as a flotation, bleaching or magnetic separation step) to remove impurities.

"Precipitated calcium carbonate" (PCC) in the meaning of the present invention is a synthesized material, generally obtained by precipitation following reaction of carbon dioxide and calcium hydroxide in an aqueous environment or by precipitation of calcium and carbonate ions, for example CaCl₂ and Na₂CO₃, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the precipitated calcium carbonate is precipitated calcium carbonate, preferably comprising aragonitic, vateritic or calcitic mineralogical crystal forms or mixtures thereof.

Precipitated calcium carbonate may be ground prior to the treatment with carbon dioxide and at least one H₃O⁺ ion donor by the same means as used for grinding natural calcium carbonate as described above.

According to one embodiment of the present invention, the natural ground calcium carbonate or precipitated calcium carbonate is in form of particles having a weight median particle size *d*₅₀ of 0.05 to 10.0 µm, preferably 0.2 to 5.0 µm, and most preferably 0.4 to 3.0 µm. According to a further embodiment of the present invention, the natural ground calcium carbonate or precipitated calcium carbonate is in form of particles having a weight top cut particle size *d*₉₈ of 0.15 to 30 µm, preferably 0.6 to 15 µm, more preferably 1.2 to 10 µm, most preferably 1.5 to 4 µm, especially 1.6 µm.

The natural ground calcium carbonate and/or precipitated calcium carbonate may be used dry or suspended in water. Preferably, a corresponding slurry has a content of natural ground calcium carbonate or precipitated calcium carbonate with from 1 wt.-% to 90 wt.-%, more preferably 3 wt.-% to 60 wt.-%, even more preferably 5 wt.-% to 40 wt.-%, and most preferably 10 wt.-% to 25 wt.-% based on the weight of the slurry.

The one or more H₃O⁺ ion donor used for the preparation of surface-reacted calcium carbonate may be any strong acid, medium-strong acid, or weak acid, or mixtures thereof, generating H₃O⁺ ions under the preparation conditions. According to the present invention, the at least one H₃O⁺ ion donor can also be an acid salt, generating H₃O⁺ ions under the preparation conditions.

According to one embodiment, the at least one H₃O⁺ ion donor is a strong acid having a pKₐ of 0 or less at 20°C.

According to another embodiment, the at least one H₃O⁺ ion donor is a medium-strong acid having a pKₐ value from 0 to 2.5 at 20°C. If the pKₐ at 20°C is 0 or less, the acid is preferably selected from sulphuric acid, hydrochloric acid, or mixtures thereof. If the pKₐ at 20°C is from 0 to 2.5, the H₃O⁺ ion donor is preferably selected from H₂SO₃, H₃PO₄, oxalic acid, or mixtures thereof. The at least one H₃O⁺ ion donor can also be an acid salt, for example, HSO₄⁻ or H₂PO₄⁻, being at least partially neutralized by a corresponding cation such as Li⁺, Na⁺ or K⁺, or HPO₄²⁻; being at least partially neutralised by a corresponding cation such as Li⁺, Na^{+,} K⁺, Mg²⁺ or Ca²⁺. The at least one H₃O⁺ ion donor can also be a mixture of one or more acids and one or more acid salts.

According to still another embodiment, the at least one H₃O⁺ ion donor is a weak acid having a pKₐ value of greater than 2.5 and less than or equal to 7, when measured at 20°C, associated with the ionisation of the first available hydrogen, and having a corresponding anion, which is capable of forming water-soluble calcium salts. Subsequently, at least one water-soluble salt, which in the case of a hydrogen-containing salt has a pKₐ of greater than 7, when measured at 20°C, associated with the ionisation of the first available hydrogen, and the salt anion of which is capable of forming water-insoluble calcium salts, is additionally provided. According to the preferred embodiment, the weak acid has a pKₐ value from greater than 2.5 to 5 at 20°C, and more preferably the weak acid is selected from the group consisting of acetic acid, formic acid, propanoic acid, and mixtures thereof. Exemplary cations of said water-soluble salt are selected from the group consisting of potassium, sodium, lithium and mixtures thereof. In a more preferred embodiment, said cation is sodium or potassium. Exemplary anions of said water-soluble salt are selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of drop wise addition, this addition preferably takes place within a time period of 10 minutes. It is more preferred to add said salt in one step.

According to one embodiment of the present invention, the at least one H₃O⁺ ion donor is selected from the group consisting of hydrochloric acid, sulphuric acid, sulphurous acid, phosphoric acid, citric acid, oxalic acid, acetic acid, formic acid, and mixtures thereof. Preferably the at least one H₃O⁺ ion donor is selected from the group consisting of hydrochloric acid, sulphuric acid, sulphurous acid, phosphoric acid, oxalic acid, H₂PO₄; being at least partially neutralised by a corresponding cation such as Li⁺, Na⁺ or K⁺, HPO₄²⁻, being at least partially neutralised by a corresponding cation such as Li⁺, Na^{+,} K⁺, Mg²⁺, or Ca²⁺ and mixtures thereof, more preferably the at least one acid is selected from the group consisting of hydrochloric acid, sulphuric acid, sulphurous acid, phosphoric acid, oxalic acid, or mixtures thereof, and most preferably, the at least one H₃O⁺ ion donor is phosphoric acid.

The one or more H₃O⁺ ion donor can be added to the suspension as a concentrated solution or a more diluted solution. Preferably, the molar ratio of the H₃O⁺ ion donor to the natural or precipitated calcium carbonate is from 0.01 to 4, more preferably from 0.02 to 2, even more preferably 0.05 to 1 and most preferably 0.1 to 0.58.

As an alternative, it is also possible to add the H₃O⁺ ion donor to the water before the natural or precipitated calcium carbonate is suspended.

In a next step, the natural ground calcium carbonate or precipitated calcium carbonate is treated with carbon dioxide. If a strong acid such as sulphuric acid or hydrochloric acid is used for the H₃O⁺ ion donor treatment of the natural ground calcium carbonate or precipitated calcium carbonate, the carbon dioxide is automatically formed. Alternatively or additionally, the carbon dioxide can be supplied from an external source.

H₃O⁺ ion donor treatment and treatment with carbon dioxide can be carried out simultaneously which is the case when a strong or medium-strong acid is used. It is also possible to carry out H₃O⁺ ion donor treatment first, e.g. with a medium strong acid having a pKₐ from 0 to 2.5 at 20°C, wherein carbon dioxide is formed in situ, and thus, the carbon dioxide treatment will automatically be carried out simultaneously with the H₃O⁺ ion donor treatment, followed by the additional treatment with carbon dioxide supplied from an external source.

In a preferred embodiment, the H₃O⁺ ion donor treatment step and/or the carbon dioxide treatment step are repeated at least once, more preferably several times. According to one embodiment, the at least one H₃O⁺ ion donor is added over a time period of at least about 5 min, typically from about 5 to about 30 min. Alternatively, the at least one H₃O⁺ ion donor is added over a time period of about 30 min, preferably about 45 min, and sometimes about 1 h or more.

Subsequent to the H₃O⁺ ion donor treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the surface-reacted natural or precipitated calcium carbonate as an aqueous suspension having a pH of greater than 6.0, preferably greater than 6.5, more preferably greater than 7.0, even more preferably greater than 7.5.

It is appreciated that the H₃O⁺ ion donor treatment and treatment with carbon dioxide can be carried over a wide temperature range. Preferably, the H₃O⁺ ion donor treatment and treatment with carbon dioxide can be carried out at room temperature or elevated temperature. For example, if the H₃O⁺ ion donor treatment and treatment with carbon dioxide is carried out at elevated temperature, the treatment is preferably in a range from 30 to 90 °C, more preferably from 40 to 80 °C and most preferably from 50 to 80 °C, such as from 60 to 80 °C.

Further details about the preparation of the surface-reacted natural calcium carbonate are disclosed in WO0039222 A1, WO2004083316 A1, WO2005121257 A2, WO2009074492 A1, EP2264108 A1, EP2264109 A1 and US20040020410 A1, the content of these references herewith being included in the present application.

Similarly, surface-reacted precipitated calcium carbonate is obtained. As can be taken in detail from WO2009074492 A1, surface-reacted precipitated calcium carbonate is obtained by contacting precipitated calcium carbonate with H₃O⁺ ions and with anions being solubilized in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by H₃O⁺ ions, where said H₃O⁺ ions are provided solely in the form of a counterion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in situ.

Said H₃O⁺ ions may be provided by the addition of an acid or an acid salt of said anion, or the addition of an acid or an acid salt which simultaneously serves to provide all or part of said excess solubilized calcium ions.

In a further preferred embodiment of the preparation of the surface-reacted natural ground calcium carbonate or precipitated calcium carbonate, the natural ground calcium carbonate or precipitated calcium carbonate is reacted with the acid and/or the carbon dioxide in the presence of at least one compound selected from the group consisting of silicate, silica, aluminium hydroxide, earth alkali aluminate such as sodium or potassium aluminate, magnesium oxide, or mixtures thereof. Preferably, the at least one silicate is selected from an aluminium silicate, a calcium silicate, or an earth alkali metallic silicate. These components can be added to an aqueous suspension comprising the natural ground calcium carbonate or precipitated calcium carbonate before adding the acid and/or carbon dioxide.

Alternatively, the silicate and/or silica and/or aluminium hydroxide and/or earth alkali aluminate and/or magnesium oxide component(s) can be added to the aqueous suspension of natural or precipitated calcium carbonate while the reaction of natural or precipitated calcium carbonate with an acid and carbon dioxide has already started. Further details about the preparation of the surface-reacted natural or precipitated calcium carbonate in the presence of at least one silicate and/or silica and/or aluminium hydroxide and/or earth alkali aluminate component(s) are disclosed in WO2004083316 A1, the content of this reference herewith being included in the present application.

The surface-reacted calcium carbonate can be kept in suspension, optionally further stabilised by a dispersant. Conventional dispersants known to the skilled person can be used. A preferred dispersant is comprised of polyacrylic acids and/or carboxymethylcelluloses.

Alternatively, the aqueous suspension described above can be dried, thereby obtaining the solid (i.e. dry or containing as little water that it is not in a fluid form) surface-reacted natural ground calcium carbonate or precipitated calcium carbonate in the form of granules or a powder.

The surface-reacted calcium carbonate may have different particle shapes, such as e.g. the shape of roses, golf balls and/or brains.

According to one embodiment, the surface-reacted calcium carbonate has a specific surface area of from 15 m²/g to 200 m²/g, preferably from 20 m²/g to 180 m²/g, and more preferably from 25 m²/g to 160 m²/g, and most preferably from 30 m²/g to 90 m²/g measured using nitrogen and the BET method. The BET specific surface area in the meaning of the present invention is defined as the surface area of the particles divided by the mass of the particles. As used therein the specific surface area is measured by adsorption using the BET isotherm (ISO 9277:2010) and is specified in m²/g.

It has surprisingly been found by the inventors that the use of a surface-reacted calcium carbonate having a specific surface area from 30 m²/g to 90 m²/g as the second component of the inventive dry cosmetic and/or skin care composition results in a dry cosmetic and/or skin care composition, which has specifically good sensory properties such as skin adherence, softness, spreadability etc.

According to one embodiment the surface-reacted calcium carbonate has a volume median particle size *d*₅₀ from 0.1 to 75 µm, preferably from 0.5 to 50 µm, more preferably from 1 to 40 µm, even more preferably from 1.2 to 30 µm, even more preferably from 1.5 to 15 µm, and most preferably from 3 to 10 µm.

It may furthermore be preferred that the surface-reacted calcium carbonate particles have a volume top cut particle size *d*₉₈ of from 2 to 150 µm, preferably from 4 to 100 µm, more preferably 6 to 80 µm, even more preferably from 8 to 60 µm, even more preferably from 8 to 30 µm, and most preferably from 12 to 25 µm.

The value *dₓ* represents the diameter relative to which x % of the particles have diameters less than *dₓ*. This means that the *d*₉₈ value is the particle size at which 98 % of all particles are smaller. The *d*₉₈ value is also designated as "top cut". The d*ₓ* values may be given in volume or weight percent. The *d*₅₀(wt) value is thus the weight median particle size, i.e. 50 wt.-% of all grains are smaller than this particle size, and the *d*₅₀ (vol) value is the volume median particle size, i.e. 50 vol.% of all grains are smaller than this particle size.

Volume median grain diameter *d*₅₀ was evaluated using a Malvern Mastersizer 2000 Laser Diffraction System or a Malvern Mastersizer 3000 Laser Diffraction System. The *d*₅₀ or *d*₉₈ value, measured using a Malvern Mastersizer 2000 Laser Diffraction System or a Malvern Mastersizer 3000 Laser Diffraction System, indicates a diameter value such that 50 % or 98 % by volume, respectively, of the particles have a diameter of less than this value. The raw data obtained by the measurement are analysed using the Mie theory, with a particle refractive index of 1.57 and an absorption index of 0.005. The measurements were carried out in an aqueous solution of 0.1 wt.-% Na₄P₂O₇.

The weight median grain diameter is determined by the sedimentation method, which is an analysis of sedimentation behaviour in a gravimetric field. The measurement is made with a Sedigraph^{™} 5100 or 5120, Micromeritics Instrument Corporation. The method and the instrument are known to the skilled person and are commonly used to determine grain size of fillers and pigments. The measurement is carried out in an aqueous solution of 0.1 wt.-% Na₄P₂O₇. The samples were dispersed using a high speed stirrer and sonicated.

The processes and instruments are known to the skilled person and are commonly used to determine grain size of fillers and pigments.

The specific pore volume is measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 µm (~ nm). The equilibration time used at each pressure step is 20 seconds. The sample material is sealed in a 5 cm³ chamber powder penetrometer for analysis. The data are corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p. 1753-1764).

The total pore volume seen in the cumulative intrusion data can be separated into two regions with the intrusion data from 214 µm down to about 1 - 4 µm showing the coarse packing of the sample between any agglomerate structures contributing strongly. Below these diameters lies the fine interparticle packing of the particles themselves. If they also have intra particle pores, then this region appears bi-modal, and by taking the specific pore volume intruded by mercury into pores finer than the modal turning point, i.e. finer than the bi-modal point of inflection, the specific intraparticle pore volume is defined. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired pore volume of the internal pores alone in terms of the pore volume per unit mass (specific pore volume). The same principle of subtraction, of course, applies for isolating any of the other pore size regions of interest.

Preferably, the surface-reacted calcium carbonate has an intra-particle intruded specific pore volume in the range from 0.1 to 2.3 cm³/g, more preferably from 0.2 to 2.0 cm³/g, especially preferably from 0.4 to 1.8 cm³/g and most preferably from 0.6 to 1.6 cm³/g, calculated from mercury porosimetry measurement.

The intra-particle pore size of the surface-reacted calcium carbonate preferably is in a range of from 0.004 to 1.6 µm, more preferably in a range of between 0.005 to 1.3 µm, especially preferably from 0.006 to 1.15 µm and most preferably of 0.007 to 1.0 µm, e.g. 0.004 to 0.16 µm determined by mercury porosimetry measurement.

According to an exemplary embodiment, the surface-reacted calcium carbonate has a volume median particle size *d*₅₀ from 1.5 to 15 µm, preferably from 4 to 8 µm; a specific surface-area of from 30 to 140 m²/g, preferably from 30 to 90 m²/g, measured using nitrogen and the BET method; and an intra-particle intruded specific pore volume from 0.2 to 2.0 cm³/g, preferably from 0.6 to 1.6 cm³/g, calculated from mercury porosimetry measurement.

According to another exemplary embodiment, the surface-reacted calcium carbonate has a volume median particle size *d*₅₀ from 5 to 9 µm; a specific surface-area of from 45 to 85 m²/g, measured using nitrogen and the BET method; and a volume top cut particle size *d*₉₈ of from 13 to 20 µm.

Due to the intra and interpore structure of the surface-reacted calcium carbonate, it can be a superior agent to deliver previously adsorbed and/or absorbed materials over time relative to common materials having similar specific surface areas. Thus, generally, any agent fitting into the intra- and/or inter particle pores of the surface-reacted calcium carbonate is suitable to be transported by the surface- reacted calcium carbonate according to the invention. For example, active agents such as those selected from the group comprising pharmaceutically active agents, biologically active agents, disinfecting agents, preservatives, flavouring agents, surfactants, oils, fragrances, essential oils, and mixtures thereof can be used. According to one embodiment, at least one active agent is associated with the surface-reacted calcium carbonate.

According to one embodiment of the present invention, the surface-reacted calcium carbonate comprises an water-insoluble, at least partially crystalline calcium salt of an anion of the at least one acid, which is formed on the surface of the natural ground calcium carbonate or precipitated calcium carbonate. According to one embodiment, the water-insoluble, at least partially crystalline salt of an anion of the at least one acid covers the surface of the natural ground calcium carbonate or precipitated calcium carbonate at least partially, preferably completely. Depending on the employed at least one acid, the anion may be sulphate, sulphite, phosphate, citrate, oxalate, acetate, formiate and/or chloride.

According to one embodiment the surface-reacted calcium carbonate comprises:
(i) a specific surface area of from 15 to 200 m²/g measured using nitrogen and the BET method according to ISO 9277:2010, and
(ii) an intra-particle intruded specific pore volume from from 0.1 to 2.3 cm³/g calculated from mercury porosimetry measurement.

According to one preferred embodiment, the second component being a surface-reacted calcium carbonate has a specific surface area of from 20 m²/g to 180 m²/g, preferably from 25 m²/g to 160 m²/g, and most preferably from 30 m²/g to 90 m²/g measured using nitrogen and the BET method, a volume median particle size *d*₅₀ from 1.2 to 30 µm, preferably from 1.5 to 15 µm, and most preferably from 3 to 10 µm, and a volume top cut particle size *d*₉₈ of from 8 to 60 µm, even more preferably from 8 to 30 µm, and most preferably from 12 to 25 µm.

### The dry cosmetic and/or skin care composition

The present invention refers to a dry cosmetic and/or skin care composition comprising a mixture of a first component being a natural ground calcium carbonate, and a second component being a surface-reacted calcium carbonate. The surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source. The dry cosmetic and/or skin care composition contains a water content of less than 15 wt.%, based on the total weight of the cosmetic and/or skin care composition.

According to one embodiment, the dry cosmetic and/or skin care composition is a dry cosmetic composition. For example, the dry cosmetic composition may be a cosmetic powder including eyeshadow, powder makeup, lip powder, face powder, body powder or blusher. According to another embodiment, the dry cosmetic and/or skin care composition is a dry skin care composition. For example, the dry skin care composition may be a skin care powder including shaving powder, body powder, baby powder, foot powder and a deodorant powder. According to yet another embodiment, the dry cosmetic and/or skin care composition is a dry cosmetic and skin care composition.

The dry cosmetic and/or skin care composition may be present in any form, which allows its use as a dry cosmetic and/or skin care composition. The dry cosmetic and/or skin care composition may be a powder, for example, a loose powder or a compact powder. A "compact powder" in the meaning of the present invention refers to a dry cosmetic and/or skin care composition, which is prepared by adding a binder, preferably a metallic stearate, to the composition, and/or by compressing the dry composition, preferably by mechanical means, whereby a cake is formed having sufficient hardness to sustain its shape and resist crumbling. A "loose powder" in the meaning of the present invention refers to a dry cosmetic and/or skin care composition, which does not comprise a binder for preparing a compacted powder, and/or which has not been compressed to form a compact powder.

According to one embodiment, the dry cosmetic and/or skin care composition is a powder. According to one embodiment, the dry cosmetic and/or skin care composition is a loose powder or a compact powder, and preferably a loose powder. In one preferred embodiment, the dry cosmetic and/or skin care composition is a baby powder or a body powder. A "baby powder" is a powder which is usually used to absorb moisture and/or body fluids after changing a diaper, and/or to prevent diaper rash or the formation of unpleasant odors. A "body powder" is a powder which is used, for example, to absorb moisture and/or body fluids such as sweat under the armpits or at the feet to provide a dry and pleasant body feeling, to avoid the formation of unpleasant odors, and/or to avoid skin friction with e.g. clothing. Usually, a baby powder may also be used for the purposes of the body powder, and vice versa.

The dry cosmetic and/or skin care composition may comprise the first component and the second component in specific weight amounts with respect to each other. According to one embodiment, the first component is present in an amount of from 1 wt.% to 99 wt.%, preferably from 30 wt.% to 99 wt.%, more preferably from 50 wt.% to 95 wt.%, even more preferably from 60 wt.% to 95 wt.%, and most preferably from 70 wt.% to 90 wt.%, based on the total weight of the first component and the second component, and the second component is present in an amount of from 1 wt.% to 99 wt.%, preferably from 1 wt.% to 70 wt.%, more preferably from 5 wt.% to 50 wt.%, even more preferably from 5 wt.% to 40 wt.%, and most preferably from 10 wt.% to 30 wt.%, based on the total weight of the first component and the second component.

According to another preferred embodiment, the first component is present in an amount of from 85 wt.% to 95 wt.%, based on the total weight of the first component and the second component, and the second component is present in an amount of from 5 wt.% to 15 wt.%, based on the total weight of the first component and the second component.

The dry cosmetic and/or skin care composition may also comprise the first component and the second component in specific weight amounts with respect to the total weight of the dry cosmetic and/or skin care composition. According to one embodiment, the first component is present in an amount of from 1 wt.% to 99 wt.%, preferably from 30 wt.% to 99 wt.%, more preferably from 50 wt.% to 95 wt.%, even more preferably from 60 wt.% to 95 wt.%, and most preferably from 70 wt.% to 90 wt.%, based on the total weight of the dry cosmetic and/or skin care composition. According to another embodiment, the second component is present in an amount of from 1 wt.% to 99 wt.%, preferably from 1 wt.% to 70 wt.%, more preferably from 5 wt.% to 50 wt.%, even more preferably from 5 wt.% to 40 wt.%, and most preferably from 10 wt.% to 30 wt.%, based on the total weight of the dry cosmetic and/or skin care composition. According to yet another embodiment, the first component is present in an amount of from 1 wt.% to 99 wt.%, preferably from 30 wt.% to 99 wt.%, more preferably from 50 wt.% to 95 wt.%, even more preferably from 60 wt.% to 95 wt.%, and most preferably from 70 wt.% to 90 wt.%, based on the total weight of the dry cosmetic and/or skin care composition, and the second component is present in an amount of from 1 wt.% to 99 wt.%, preferably from 1 wt.% to 70 wt.%, more preferably from 5 wt.% to 50 wt.%, even more preferably from 5 wt.% to 40 wt.%, and most preferably from 10 wt.% to 30 wt.%, based on the total weight of the dry cosmetic and/or skin care composition.

For example, the first component is present in an amount of from 50 wt.% to 90 wt.%, preferably from 55 wt.% to 85 wt.%, and the second component is present in an amount from 5 wt.% to 35 wt.%, preferably from 5 wt.% to 30 wt.%, based on the total weight of the dry cosmetic and/or skin care composition. In another exemplary embodiment, the first component is present in an amount of from 60 to 90 wt.%, preferably from 65 to 85 wt.%, and the second component is present in an amount from 5 wt.% to 35 wt.%, preferably from 5 wt.% to 30 wt.%, based on the total weight of the dry cosmetic and/or skin care composition.

Furthermore, the dry cosmetic and/or skin care composition may comprise the mixture of the first component and the second component in specific weight amounts with respect to the overall weight of the composition. According to one embodiment, the dry cosmetic and/or skin care composition comprises the mixture of the first component and the second component in an amount of from 1 to 99 wt.%, preferably of from 10 to 99 wt.%, more preferably of from 30 to 98 wt.%, even more preferably of from 50 to 95 wt.%, and most preferably of from 60 to 90 wt.%, based on the total weight of the dry cosmetic and/or skin care composition.

As described above, certain more specific components may be preferred as the first component and the second component of the dry cosmetic and/or skin care composition.

According to a preferred embodiment, the dry cosmetic and/or skin care composition comprises a mixture of a first component being a natural ground calcium carbonate, and a second component being a surface-reacted calcium carbonate, wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source. According to another preferred embodiment, the dry cosmetic and/or skin care composition comprises a mixture of a first component being a natural ground calcium carbonate, and a second component being a surface-reacted calcium carbonate, wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, wherein the at least one H₃O⁺ ion donors is phosphoric acid.

According to a preferred embodiment, the dry cosmetic and/or skin care composition comprises a mixture of a first component being a natural ground calcium carbonate, and a second component being a surface-reacted calcium carbonate, wherein the first component has a volume median particle size d₅₀ from 0.5 to 10 µm, preferably from 0.8 to 8 µm, and a specific surface area of from 1 m²/g to 20 m²/g, preferably from 2 m²/g to 15 m²/g, measured using nitrogen and the BET method, and wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, wherein the at least one H₃O⁺ ion donors is phosphoric acid, and wherein the second component has a volume median particle size d₅₀ of from 1.5 to 15 µm, preferably from 3 to 10 µm, and a specific surface area of from 25 m²/g to 160 m²/g, preferably from 30 m²/g to 90 m²/g, measured using nitrogen and the BET method.

According to another preferred embodiment, the dry cosmetic and/or skin care composition comprises a mixture of a first component being a natural ground calcium carbonate, and a second component being a surface-reacted calcium carbonate, wherein the first component has a volume median particle size d₅₀ from 0.5 to 10 µm, preferably from 0.8 to 8 µm, and a specific surface area of from 1 m²/g to 20 m²/g, preferably from 2 m²/g to 15 m²/g, measured using nitrogen and the BET method, and wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, wherein the at least one H₃O⁺ ion donors is phosphoric acid, and wherein the second component has a volume median particle size d₅₀ of from 1.5 to 15 µm, preferably from 3 to 10 µm, and a specific surface area of from 25 m²/g to 160 m²/g, preferably from 30 m²/g to 90 m²/g, measured using nitrogen and the BET method, wherein the first component is present in an amount of from 50 wt.% to 95 wt.%, preferably from 60 to 95 wt.%, based on the total weight of the first component and the second component, and the second component is present in an amount of from 5 wt.% to 50 wt.%, preferably from 5 wt.% to 40 wt.%, based on the total weight of the first component and the second component, and wherein the mixture of the first component and the second component is present in an amount of from 60 to 95 wt.%, preferably of from 70 to 90 wt.%, based on the total weight of the dry cosmetic and/or skin care composition.

According to yet another preferred embodiment, the dry cosmetic and/or skin care composition comprises a mixture of a first component being a natural ground calcium carbonate, and a second component being a surface-reacted calcium carbonate, wherein the first component has a volume median particle size d₅₀ from 0.5 to 10 µm, preferably from 0.8 to 8 µm, and a specific surface area of from 1 m²/g to 20 m²/g, preferably from 2 m²/g to 15 m²/g, measured using nitrogen and the BET method, and wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, wherein the at least one H₃O⁺ ion donors is phosphoric acid, and wherein the second component has a volume median particle size d₅₀ of from 1.5 to 15 µm, preferably from 3 to 10 µm, and a specific surface area of from 25 m²/g to 160 m²/g, preferably from 30 m²/g to 90 m²/g, measured using nitrogen and the BET method, and wherein the first component is present in an amount of from 50 wt.% to 95 wt.%, preferably from 60 to 95 wt.%, based on the total weight of the first component and the second component, and the second component is present in an amount of from 5 wt.% to 50 wt.%, preferably from 5 wt.% to 40 wt.%, based on the total weight of the first component and the second component.

The dry cosmetic and/or skin care composition of the present invention preferably does not contain certain materials or components. According to one embodiment, the dry cosmetic and/or skin care composition is free of talc or a talc-containing material. A "talc or a talc-containing material" are known to the skilled person. "Talc" refers to a clay mineral comprising a hydrated magnesium silicate, which may be described by the chemical formula Mg₃Si₄O₁₀(OH)₂. Talc has a value of 1 on the Mohs hardness scale. A "talc-containing material" refers to every material, which contains talc as a component. A "talc-containing material" is, for example, ultramafic rock such as soapstone.

Talc is a common ingredient of dry cosmetic and/or skin care compositions, in which it is used for providing the dry cosmetic and/or skin care composition with specific characteristics such as humidity absorbing characteristics and a soft touch. The inventors surprisingly found that the dry cosmetic and/or skin care composition of the present invention shows similar, or even improved, characteristics, compared to known talc-containing dry cosmetic and/or skin care compositions.

According to another embodiment, the dry cosmetic and/or skin care composition does not contain a silicate- and/or aluminate-containing material. "Silicate- and/or aluminate-containing material" are known to the skilled person and may refer to, for example, mica, kaolin or silica.

The dry cosmetic and/or skin care composition may consist of a mixture of a first component and a second component as described above. However, in most cases, the dry cosmetic and/or skin care composition according to the present invention may comprise further components in addition to the mixture of the first component and the second component as described herein. The skilled person knows materials, which are allowed and/or suitable for use in a dry cosmetic and/or skin care composition, and will select such materials according to the form of the composition and its application.

According to one embodiment, the dry cosmetic and/or skin care composition comprises one or more of a further component, preferably the one or more further component is selected from the group consisting of a fragrance, an aroma, an antibacterial and/or an antiseptic agent, a fatty acid or a salt thereof, a fatty alcohol, a vegetable or a synthetic oil, a polymeric carbohydrate, a mineral additive, a pigment, a salt and mixtures thereof. It is to be understood that each optional further component may also be present a mixture of such components, i.e. two or more of such a component, unless otherwise stated. For example, a fragrance refers to one fragrance, but also encompasses a mixture of two or more fragrances.

According to one embodiment, the dry cosmetic and/or skin care composition comprises one or more of a further component in an amount of from 0.1 to 40 wt.%, preferably from 0.1 to 35 wt.%, more preferably from 10 to 35 wt.%, based on the total weight of the dry cosmetic and/or skin care composition.

The dry cosmetic and/or skin care composition may further comprise an antibacterial and/or an antiseptic agent. According to one embodiment, dry cosmetic and/or skin care composition may further comprise an antibacterial and/or an antiseptic agent further comprises an antibacterial and/or an antiseptic agent. For example, suitable antibacterial and/or antiseptic agents may be para-oxybenzoic acid alkyl ester, benzoic acid, sodium benzoate, sorbic acid, a potassium sorbate, phenoxy ethanol, salicylic acid, carbolic acid, sorbic acid, hexachlorophene, benzalkonium chloride, chlorhexidine chloride, trichlorocarbanilide, or phenoxyethanol.

For example, polymeric carbohydrates such as starch, in particular rice or corn starch, may be added to the dry cosmetic and/or skin care composition in order to further improve the application properties such as lipid absorption, spreadability or texture. According to one embodiment, the dry cosmetic and/or skin care composition further comprises a polymeric carbohydrate, preferably a natural or a modified starch, more preferably a natural starch, even more preferably a natural starch selected from the group consisting of cornstarch, rice starch or tapioca starch, or a mixture thereof. According to one preferred embodiment, the polymeric carbohydrate, preferably the starch, is present in the dry cosmetic and/or skin care composition in an amount of from 1 to 20 wt.%, preferably from 5 to 20 wt.%, more preferably from 5 to 15 wt.%, based on the total weight of the dry cosmetic and/or skin care composition.

Fatty acids or salts thereof may also be added to the dry cosmetic and/or skin care composition according to the present invention. The addition of fatty acids or salts thereof may further improve the skin adherence of the dry cosmetic and/or skin care composition and/or the compressibility of the powder. A salt derived from an organic carboxylic acid having 8 to 22 carbon atoms, preferably 12 to 18 carbon atoms, such as a metallic stearate, a metallic laurate or a metallic myristate, may be added to the inventive composition. For example, suitable salts of fatty acids may be selected from the group consisting of zinc, magnesium or lithium stearate, zinc laurate, or magnesium myristate. Thus, according to one embodiment, the dry cosmetic and/or skin care composition further comprises at least one fatty acid or a salt thereof, preferably a salt of a fatty acid, more preferably a metallic stearate, even more preferably a metallic stearate selected from the group consisting of zinc stearate, magnesium stearate, lithium stearate, or mixtures thereof. According to a preferred embodiment, the dry cosmetic and/or skin care composition further comprises at least one fatty acid or salt thereof, preferably salt of a fatty acid, more preferably a metallic stearate, in an amount of from 0.1 to 4 wt.%, preferably from 0.5 to 3 wt.%, and more preferably from 1.0 to 2.5 wt.%, based on the total weight of the dry cosmetic and/or skin care composition.

The dry cosmetic and/or skin care composition may further comprise a vegetable oil or a synthetic oil to further improve the skin adherence of the composition and/or to further increase the mildness of the application and/or to serve as a vehicle for the further components of the dry cosmetic and/or skin care composition described herein. According to another embodiment, the dry cosmetic and/or skin care composition further comprises a vegetable oil or a synthetic oil. Preferably, the dry cosmetic and/or skin care composition comprises a vegetable oil. Suitable vegetable or synthetic oils for use in dry cosmetic and/or skin care compositions are known to the skilled person. Suitable vegetable or synthetic oils are, for example, sesame oil, macadamia oil, jojoba oil, aloe vera oil or olive oil. According to a preferred embodiment, the dry cosmetic and/or skin care composition further comprises a vegetable oil, preferably olive oil, in an amount of from 0.1 to 5 wt.%, preferably from 0.5 to 4 wt.%, and more preferably from 1 to 3 wt.%, based on the total weight of the dry cosmetic and/or skin care composition.

Furthermore, the inventive dry cosmetic and/or skin care composition may further comprise a fatty alcohol, preferably a straight-chain fatty alcohol having between 8 and 22 carbon atoms. Suitable fatty alcohols may be, for example, cetyl alcohol, myristyl alcohol, stearyl alcohol, behenyl alcohol, or mixtures thereof. According to one embodiment, the dry cosmetic and/or skin care composition comprises a fatty alcohol, preferably a straight-chain fatty alcohol having between 8 and 22 carbon atoms. According to a preferred embodiment, the dry cosmetic and/or skin care composition comprises a fatty alcohol, preferably a straight-chain fatty alcohol having between 8 and 22 carbon atoms, in an amount of from 0.1 to 4 wt.%, preferably from 0.2 to 2 wt.%, and more preferably from 0.5 to 1.5 wt.%, based on the total weight of the dry cosmetic and/or skin care composition.

Further, the dry cosmetic and/or skin care composition may further comprise one or more pigments in order to further modify the skin appearance, if desired. Thus, according to one embodiment, the dry cosmetic and/or skin care composition further comprises a pigment. For example, a suitable pigment may be zinc oxide, titanium oxide, yellow iron oxide, red iron oxide, black iron oxide, a vanadium oxide, zirconium oxide or a manganese oxide. According to one embodiment, the dry cosmetic and/or skin care composition further comprises a pigment.

Additionally or alternatively, the dry cosmetic and/or skin care composition of the present invention may further comprise a mineral additive and/or a salt to further improve, for example, the absorption power, the skin adherence and/or the sun protection of the dry cosmetic and/or skin care composition. According to one embodiment, the dry cosmetic and/or skin care composition further comprises a mineral additive, preferably kaolin. According to another embodiment, the dry cosmetic and/or skin care composition further comprises a salt, preferably zinc oxide. According to yet another embodiment, the dry cosmetic and/or skin care composition further comprises a mineral additive, preferably kaolin, and a salt, preferably, zinc oxide. According to a preferred embodiment, the dry cosmetic and/or skin care composition further comprises a mineral additive, preferably kaolin, in an amount of from 0.1 to 7.5 wt.%, preferably from 2 to 7.5 wt.%, and more preferably from 4 to 6 wt.%, based on the total weight of the dry cosmetic and/or skin care composition. According to another preferred embodiment, the dry cosmetic and/or skin care composition further comprises a salt, preferably zinc oxide, in an amount of from 0.1 to 5 wt.%, preferably from 1 to 4 wt.%, and more preferably from 2 to 3 wt.%, based on the total weight of the dry cosmetic and/or skin care composition.

Moreover, the dry cosmetic and/or skin care composition according to the present invention may comprise a fragrance and/or an aroma to further improve the odor of the composition before, during and/or after the application onto the skin and/or to provide the skin with a more pleasant odor during and/or after application of the composition onto the skin. Thus, according to one embodiment, the dry cosmetic and/or skin care composition further comprises a fragrance and/or an aroma. The fragrance and/or the aroma may be adsorbed and/or absorbed into the surface of the first component and/or to the surface of the second component being present in the dry cosmetic and/or skin care composition. According to one embodiment, the fragrance and/or the aroma is adsorbed and/or absorbed into the surface of the first component and/or to the surface of the second component.

A "fragrance" in the meaning of the present invention refers to a natural or synthetic organic compound, which is sufficiently volatile to be perceived by a person via the olfactory system. The fragrance may be selected from a natural and/or synthetic fragrance known to being suitable in cosmetic formulations, for example, such as mint oil. In one embodiment, the dry cosmetic and/or skin care composition further comprises a fragrance in an amount of from 0.01 to 5.0 wt.%, preferably from 0.5 to 3.0 wt.%, and more preferably from 1.0 to 2.0 wt.%, based on the total weight of the dry cosmetic and/or skin care composition.

An "aroma" in the meaning of the present invention refers to one or more natural or synthetic organic compounds, which provide a composition with a specific scent and taste. The aroma may be selected from ketones, esters, aldehydes, terpenes, aromatics, alkylpyrazines or mixtures thereof. A natural aroma is, for example, vanillin. A synthetic aroma is, for example, ethylvanillin. The aroma may also refer to an aroma extract obtained from fruits, seeds or herbs. For example, the aroma may be a vanilla or a strawberry extract. In one embodiment, the dry cosmetic and/or skin care composition further comprises an aroma in an amount of from 0.01 to 5.0 wt.%, preferably from 0.5 to 3.0 wt.%, and more preferably from 1.0 to 2.0 wt.%, based on the total weight of the dry cosmetic and/or skin care composition.

According to one embodiment, the dry cosmetic and/or skin care composition further comprises a polymeric carbohydrate, preferably a starch, in an amount of from 1 to 20 wt.%, preferably from 5 to 15 wt.%, a fatty acid or a salt thereof, preferably a metallic stearate, in an amount of from 0.1 to 4 wt.%, and more preferably from 1.0 to 2.5 wt.%, a vegetable oil, preferably olive oil, in an amount of from 0.1 to 5 wt.%, preferably from 1 to 3 wt.%, a fatty alcohol, preferably a straight-chain fatty alcohol having between 8 and 22 carbon atoms, in an amount of from 0.1 to 4 wt.%, preferably from 0.5 to 1.5 wt.%, a mineral additive, preferably kaolin, in an amount of from 0.1 to 7.5 wt.%, preferably from 4 to 6 wt.%, and a salt, preferably zinc oxide, in an amount of from 0.1 to 5 wt.%, preferably from 2 to 3 wt.%, based on the total weight of the dry cosmetic and/or skin care composition.

According to one embodiment, the dry cosmetic and/or skin care composition comprises a first component as defined herein in an amount of 50 to 90 wt.%, preferably 60 to 90 wt.%, a second component as defined herein in an amount of 5 to 50 wt.%, preferably 5 to 40 wt.%, a polymeric carbohydrate, preferably a starch, in an amount of from 1 to 20 wt.%, preferably from 5 to 15 wt.%, a fatty acid or a salt thereof, preferably a metallic stearate, in an amount of from 0.1 to 4 wt.%, and more preferably from 1.0 to 2.5 wt.%, a vegetable oil, preferably olive oil, in an amount of from 0.1 to 5 wt.%, preferably from 1 to 3 wt.%, a fatty alcohol, preferably a straight-chain fatty alcohol having between 8 and 22 carbon atoms, in an amount of from 0.1 to 4 wt.%, preferably from 0.5 to 1.5 wt.%, a mineral additive, preferably kaolin, in an amount of from 0.1 to 7.5 wt.%, preferably from 4 to 6 wt.%, and a salt, preferably zinc oxide, in an amount of from 0.1 to 5 wt.%, preferably from 2 to 3 wt.%, based on the total weight of the dry cosmetic and/or skin care composition.

According to one embodiment, the dry cosmetic and/or skin care composition comprises the mixture of the first component and the second component in an amount of 50 to 95 wt.%, preferably 60 to 90 wt.%, a polymeric carbohydrate, preferably a starch, in an amount of from 1 to 20 wt.%, preferably from 5 to 15 wt.%, a fatty acid or a salt thereof, preferably a metallic stearate, in an amount of from 0.1 to 4 wt.%, and more preferably from 1.0 to 2.5 wt.%, a vegetable oil, preferably olive oil, in an amount of from 0.1 to 5 wt.%, preferably from 1 to 3 wt.%, a fatty alcohol, preferably a straight-chain fatty alcohol having between 8 and 22 carbon atoms, in an amount of from 0.1 to 4 wt.%, preferably from 0.5 to 1.5 wt.%, a mineral additive, preferably kaolin, in an amount of from 0.1 to 7.5 wt.%, preferably from 4 to 6 wt.%, and a salt, preferably zinc oxide, in an amount of from 0.1 to 5 wt.%, preferably from 2 to 3 wt.%, a fragrance in an amount of from 0.01 to 5.0 wt.%, preferably from 0.5 to 3.0 wt.%, based on the total weight of the dry cosmetic and/or skin care composition, and wherein the dry cosmetic and/or skin care composition comprises the first component in an amount of from 60 to 95 wt.%, preferably of from 70 to 90 wt.%, and the second component in an amount of from 5 to 40 wt.%, preferably of from 10 to 30 wt.%, based on the total weight of the first component and the second component.

### The process

The present invention further refers to a process for preparing a dry cosmetic and/or skin care composition as defined in the appended claims. The process comprises the following steps:
a) providing a first component being a natural ground calcium carbonate,
b) providing a second component being a surface-reacted calcium carbonate, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b).

The first component of step a) may be provided in dry form or in form of an aqueous suspension. According to one embodiment, the first component of step a) is provided in dry form. Preferably, the first component provided in step a) has a water content of below 3.0 wt.%, preferably of below 2.0 wt.%, and more preferably of below 1.0 wt.%, based on the total dry weight of the first component. The residual moisture content may be determined as described above for the dry cosmetic and/or skin care composition.

According to another embodiment, the first component of step a) is provided in form of an aqueous suspension, preferably in form of a slurry. Preferably, the first component provided in step a) is present in the slurry of from 1 wt.-% to 90 wt.-%, more preferably 3 wt.-% to 60 wt.-%, even more preferably 5 wt.-% to 40 wt.-%, and most preferably 10 wt.-% to 25 wt.-% based on the weight of the slurry.

Similarly, the second component provided in step b) may also be provided in dry form or in form of an aqueous suspension. According to one embodiment, the second component of step b) is provided in dry form. Preferably, the second component has a water content of below 10 wt.%, preferably of below 5.0 wt.%, and more preferably of below 3.0 wt.%, based on the total dry weight of the second component. The residual moisture content may be determined as described above for the dry cosmetic and/or skin care composition.

In an alternative embodiment, the second component of step b) is provided in form of an aqueous suspension, preferably in form of a slurry. Preferably, the second component provided in step b) is present in the slurry of from 1 wt.-% to 90 wt.-%, more preferably 3 wt.-% to 60 wt.-%, even more preferably 5 wt.-% to 40 wt.-%, and most preferably 10 wt.-% to 25 wt.-%, based on the weight of the slurry.

The first component of step a) and the second component of step b) may be provided in dry form. Thus, according to one embodiment, the first component of step a) and the second component of step b) are provided in dry form.

However, it is also possible that the first component of step a) and/or the second component of step b) is provided in form of an aqueous suspension. Thus, according to one embodiment, the first component of step a) is provided in dry form, and the second component of step b) is provided in form of an aqueous suspension, preferably in form of a slurry. According to another embodiment, the first component of step a) is provided in form of an aqueous suspension, preferably in form of a slurry, and the second component of step b) is provided in dry form. According to yet another embodiment, the first component of step a) and the second component of step b) are provided in form of an aqueous suspension, preferably in form of a slurry. According to one embodiment, an aqueous dispersion, preferably a slurry, is obtained in mixing step c) having a solids content of from 1 to 90 wt.%, preferably from 3 to 60 wt.%, more preferably from 10 to 45 wt.%, and most preferably from 10 to 40 wt.%, based on the total weight of the aqueous dispersion obtained in mixing step c).

In case the first component of step a) and the second component of step b) are provided in dry form, mixing step c) may be a dry blending step. According to one embodiment, mixing step c) is a dry blending step. Preferably, the dry blending step is carried out with a plow share mixer, a ribbon mixer or a cone single shaft mixer, and more preferably with a cone single shaft mixer. The skilled person is familiar with such type of mixers.

The first component of step a) and the second component of step b) may be added to a mixing vessel in mixing step c), simultaneously or in any order. According to one embodiment, the first component of step a) is added to the second component of step b) to a mixing vessel in mixing step c), or vice versa. According to another embodiment, the first component of step a) and the second component of step b) are added simultaneously to a mixing vessel in mixing step c).

If mixing step c) is a dry blending step, the dry cosmetic and/or skin care composition according to the invention may be obtained directly in mixing step c). In one embodiment, the dry cosmetic and/or skin care composition according to the invention is obtained in mixing step c).

Accordingly, in one embodiment, the process for preparing a dry cosmetic and/or skin care composition comprises the following steps:
a) providing a first component being a natural ground calcium carbonate,
b) providing a second component being a surface-reacted calcium carbonate, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b) to obtain a dry cosmetic and/or skin care composition.

In one preferred embodiment, the process for preparing a dry cosmetic and/or skin care composition comprises the following steps:
a) providing a first component being a natural ground calcium carbonate in dry form,
b) providing a second component being a surface-reacted calcium carbonate in dry form, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b) to obtain a dry cosmetic and/or skin care composition, wherein mixing step c) is a dry blending step.

In case the first component of step a) and/or the second component of step b) is provided in form of an aqueous suspension, mixing step c) may be a wet mixing step. According to one embodiment, mixing step c) is a wet mixing step. A "wet mixing step" in the meaning of the present invention refers to the mixing of an aqueous suspension or a slurry. The wet mixing step may be carried out under conventional mixing conditions. The skilled man will adapt these mixing conditions (such as the configuration of mixing pallets and mixing speed) according to his process equipment. It is appreciated that any wet mixing method which would be suitable to a cosmetic and/or skin care composition may be used.

If mixing step c) is a wet mixing step, the process further comprises a drying step d) for providing the dry cosmetic and/or skin care composition according to the invention. The drying step d) may be carried out by any drying process, which is suitable for drying a cosmetic and/or skin care composition. Preferably, the drying step d) is a spray drying step. According to one embodiment, the process further comprises a drying step d), preferably a spray drying step or a superheated steam drying step, and more preferably a spray drying step. Preferably, the spray drying step is carried out at between 80 and 250°C, more preferably between 100 and 200°C, until a constant dry weight of the dry cosmetic and/or skin care composition is reached. Drying particulate matter by superheated steam drying is known to the skilled person. For example, the superheated steam drying step may be carried out as described in WO2012140028.

According to one embodiment, the process for preparing a dry cosmetic and/or skin care composition comprises the following steps:
a) providing a first component being a natural ground calcium carbonate in form of an aqueous suspension, preferably a slurry,
b) providing a second component being a surface-reacted calcium carbonate in form of an aqueous suspension, preferably a slurry, or in dry form, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b),
d) drying the mixture obtained in step c), preferably by spray drying, to obtain a dry cosmetic and/or skin care composition.

According to one embodiment, the process for preparing a dry cosmetic and/or skin care composition comprises the following steps:
a) providing a first component being a natural ground calcium carbonate in form of an aqueous suspension, preferably a slurry, or in dry form,
b) providing a second component being a surface-reacted calcium carbonate in form of an aqueous suspension, preferably a slurry, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b),
d) drying the mixture obtained in step c), preferably by spray drying, to obtain a dry cosmetic and/or skin care composition.

According to one embodiment, the process for preparing a dry cosmetic and/or skin care composition comprises the following steps:
a) providing a first component being a natural ground calcium carbonate in form of an aqueous suspension, preferably a slurry,
b) providing a second component being a surface-reacted calcium carbonate in form of an aqueous suspension, preferably a slurry, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b),
d) drying the mixture obtained in step c), preferably by spray drying, to obtain a dry cosmetic and/or skin care composition.

In one preferred embodiment, the process for preparing a dry cosmetic and/or skin care composition comprises the following steps:
a) providing a first component being a natural ground calcium carbonate,
b) providing a second component being a surface-reacted calcium carbonate, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, wherein the at least one H₃O⁺ ion donors is phosphoric acid,
c) mixing the first component of step a) with the second component of step b).

In one preferred embodiment, the process for preparing a dry cosmetic and/or skin care composition comprises the following steps:
a) providing a first component being a natural ground calcium carbonate, wherein the first component has a volume median particle size d₅₀ from 0.5 to 10 µm, preferably from 0.8 to 8 µm, and a specific surface area of from 1 m²/g to 20 m²/g, preferably from 2 m²/g to 15 m²/g, measured using nitrogen and the BET method
b) providing a second component being a surface-reacted calcium carbonate, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, wherein the at least one H₃O⁺ ion donors is phosphoric acid,
c) mixing the first component of step a) with the second component of step b).

In one preferred embodiment, the process for preparing a dry cosmetic and/or skin care composition comprises the following steps:
a) providing a first component being a natural ground calcium carbonate in dry form, wherein the first component has a volume median particle size d₅₀ from 0.5 to 10 µm, preferably from 0.8 to 8 µm, and a specific surface area of from 1 m²/g to 20 m²/g, preferably from 2 m²/g to 15 m²/g, measured using nitrogen and the BET method
b) providing a second component being a surface-reacted calcium carbonate, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, wherein the at least one H₃O⁺ ion donors is phosphoric acid, and wherein the second component has a volume median particle size d₅₀ of from 1.5 to 15 µm, preferably from 3 to 10 µm, and a specific surface area of from 25 m²/g to 160 m²/g, preferably from 30 m²/g to 90 m²/g, measured using nitrogen and the BET method,
c) mixing the first component of step a) with the second component of step b).

In one preferred embodiment, the process for preparing a dry cosmetic and/or skin care composition comprises the following steps:
a) providing a first component being a natural ground calcium carbonate in dry form, wherein the first component has a volume median particle size d₅₀ from 0.5 to 10 µm, preferably from 0.8 to 8 µm, and a specific surface area of from 1 m²/g to 20 m²/g, preferably from 2 m²/g to 15 m²/g, measured using nitrogen and the BET method
b) providing a second component being a surface-reacted calcium carbonate in dry form, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, wherein the at least one H₃O⁺ ion donors is phosphoric acid, and wherein the second component has a volume median particle size d₅₀ of from 1.5 to 15 µm, preferably from 3 to 10 µm, and a specific surface area of from 25 m²/g to 160 m²/g, preferably from 30 m²/g to 90 m²/g, measured using nitrogen and the BET method,
c) mixing the first component of step a) with the second component of step b) to obtain a dry cosmetic and/or skin care composition.

The inventive process may comprise further steps depending on the form of the dry cosmetic and/or skin care composition. For example, if the dry cosmetic and/or skin care composition is a compact powder, the process may comprise a step e) of compacting the powder into a compact powder. Preferably, such a compacting step is carried out by applying a mechanical force to the composition. In one embodiment, the process further comprises a step e) of compacting the dry cosmetic and/or skin care composition.

Furthermore, the process may further comprise a step f) of packaging the dry cosmetic and/or skin care composition. The dry cosmetic and/or skin care composition may be packaged in any container, which is suitable for storing a dry cosmetic and/or skin care composition such as a powder bottle, a powder canister, a metal pan or godet, or a cosmetic case.

In one embodiment, the process for preparing a dry cosmetic and/or skin care composition comprising the following steps:
a) providing a first component being a natural ground calcium carbonate,
b) providing a second component being a surface-reacted calcium carbonate, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b), and
f) packaging the dry cosmetic and/or skin care composition.

In one embodiment, the process for preparing a dry cosmetic and/or skin care composition comprising the following steps:
a) providing a first component being a natural ground calcium carbonate in dry form,
b) providing a second component being a surface-reacted calcium carbonate in dry form, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b) to obtain a dry cosmetic and/or skin care composition, and
f) packaging the dry cosmetic and/or skin care composition obtained in step c).

The process may further comprise a step g) of applying the dry cosmetic and/or skin care composition onto the skin to achieve one or more of the desired effects described herein such as modifying the skin feel, modifying the skin appearance or absorbing body fluids. Thus, according to one embodiment, the process further comprises a step g) of applying the dry cosmetic and/or skin care composition onto the skin of a person.

In one embodiment, the process for preparing a dry cosmetic and/or skin care composition comprising the following steps:
a) providing a first component being a natural ground calcium carbonate,
b) providing a second component being a surface-reacted calcium carbonate, wherein step b) comprises the steps of:
   step b1) reacting a natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, and optionally
   step b2) drying the reaction product obtained in step b1)
c) mixing the first component of step a) with the second component of step b), and
g) applying the dry cosmetic and/or skin care composition onto the skin of a person.

In one embodiment, the process for preparing a dry cosmetic and/or skin care composition comprising the following steps:
a) providing a first component being a natural ground calcium carbonate in dry form,
b) providing a second component being a surface-reacted calcium carbonate in dry form, wherein
   the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b) to obtain a dry cosmetic and/or skin care composition, and
g) applying the dry cosmetic and/or skin care composition onto the skin of a person.

### The uses

The present invention also refers to a use of a dry composition comprising a mixture of a first component being a natural ground calcium carbonate, and a second component being a surface-reacted calcium carbonate, wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, as a cosmetic and/or skin care composition.

The use of the dry composition as described herein as a dry cosmetic and/or skin care composition is meant to encompass the use of the dry composition in a dry cosmetic and/or skin care composition or the use of the dry composition as a component of a dry cosmetic and/or skin care composition. Thus, according to one embodiment, the use of a dry composition as described herein is used in a cosmetic and/or skin care composition. According to one embodiment, the use of a dry composition as described herein is used as a component of a cosmetic and/or skin care composition.

The use of the dry cosmetic and/or skin care composition is not limited to a specific part of the skin. For example, the dry cosmetic and/or skin care composition may be used on facial skin, skin of the hand and/or arm, skin of the foot and/or leg, skin of the genital area, skin of the buttocks, skin of the stomach and/or chest, skin of the shoulders and/or back, skin of the arm pits etc. According to one embodiment, the dry cosmetic and/or skin care composition is used on the skin, preferably on the facial skin, skin of the hand and/or arm, skin of the foot and/or leg, skin of the genital area, skin of the buttocks, skin of the stomach and/or chest, skin of the shoulders and/or back, and/or skin of the arm pits, preferably on facial skin, skin of the foot, skin of the genital area, skin of the buttocks, and/or skin of the arm pits.

The dry cosmetic and/or skin care composition of the present invention may be used for absorbing fluids, for decreasing skin friction, for modifying the skin feel, and/or for modifying the skin appearance. According to one embodiment, the dry cosmetic and/or skin care composition of the present invention is used for absorbing fluids, for decreasing skin friction, for modifying the skin feel, and/or for modifying the skin appearance. According to one preferred embodiment, the dry cosmetic and/or skin care composition of the present invention is used for absorbing fluids, for decreasing skin friction, for modifying the skin feel, and/or for modifying the skin appearance.

For example, the dry cosmetic and/or skin care composition may be used for absorbing fluids, preferably moisture and/or body fluids such as sweat, sebum, lipids or urine, to provide the user with a dry and/or more pleasant skin feeling, to avoid the formation of unpleasant odors, and/or to sooth the skin. Thus, according to one embodiment, the dry cosmetic and/or skin care composition is used for absorbing moisture and/or body fluids, preferably the body fluids are sweat, sebum, lipids or urine. According to one embodiment, the dry cosmetic and/or skin care composition is used for absorbing moisture.

The dry cosmetic and/or skin care composition may further be used for decreasing the friction between the skin and an object being in contact therewith, preferably clothing, a diaper and/or a polymeric material of e.g. a latex or nitrile glove. Thus, according to one embodiment, the dry cosmetic and/or skin care is used for decreasing the friction between the skin and an object being in contact therewith, preferably clothing, a diaper, and/or a polymeric material, preferably latex or nitrile rubber.

Furthermore, the dry cosmetic and/or skin care composition may be used to modify the skin feel, for example, by providing the user with a soft, dry and/or smooth skin feeling. According to one embodiment, the dry cosmetic and/or skin care composition is used for modifying the skin feel, preferably softening and/or smoothening the skin, drying the skin and/or soothing the skin. As described above, the expression "skin feel" in the meaning of the present invention refers to the feeling of the skin during and/or after the application of the dry cosmetic and/or skin care composition onto the skin surface.

Furthermore, the dry cosmetic and/or skin care composition may be used to modify the skin appearance. The expression "skin appearance" in the meaning of the present invention relates to the optical impression of the skin to the eye of the beholder during and/or after application of the dry cosmetic and/or skin care composition. The desired modification of the skin appearance usually depends on the type of application of the dry cosmetic and/or skin care composition, and may refer to, for example, to mattifying the skin, colouring the skin, bleaching or whitening the skin. For certain uses of the dry cosmetic and/or skin care composition such as the use as a baby powder and/or a body powder a skin bleaching and/or skin whitening effect may be desired, because it is often perceived by the user as giving the skin a particular clean and/or well-cared look.

According to one embodiment, the dry cosmetic and/or skin care composition is used for modifying the skin appearance, preferably for mattifying the skin, colouring the skin, bleaching and/or whitening the skin. In one preferred embodiment, the dry cosmetic and/or skin care composition is used for bleaching and/or whitening of the skin.

According to a preferred embodiment, the dry cosmetic and/or skin care composition is used as a cosmetic and/or skin care powder, preferably as a baby powder and/or a body powder.

The present invention further refers to the use of a mixture comprising a first component being a natural ground calcium carbonate, and a second component being a surface-reacted calcium carbonate, wherein the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source, as a replacement for talc or a talc-containing material in a dry cosmetic and/or skin care composition. Preferably, the mixture of the first and second component is used to as replacement of talc or a talc-containing material in a baby powder and/or a body powder.

The inventors surprisingly found that the mixture of the first component and a second component as described herein mimics to a certain extent the characteristics of talc as a component of a dry cosmetic and/or skin care composition such as a baby and/or body powder. In particular, it has been found that the use of a mixture of the first component and a second component as described herein in a dry cosmetic and/or skin care composition has similar, or even improved, properties as regards skin feel during and/or after application, skin appearance, moisture and body fluid absorption compared to a dry cosmetic and/or skin care composition, which contains the comparable or the same amount of talc to provide these properties.

### Figures

Fig. 1: Figure 1 shows the sensorial properties of inventive composition 1 to 4 and comparative composition 1 as evaluated by a panel of eight assessor.
Fig 2: Figure 2 shows the ad-/absorption of moisture by inventive composition 1 to 4 and comparative composition 1. The change in weight of the respective composition is plotted against the relative humidity, in which the sample was stored for 360 min by 23°C.
Fig. 3: Figure 3 refers to the overall fragrance release of inventive composition 5 to 8 and comparative composition 2 as well as comparative composition CC-Y immediately after loading of the composition with fragrance (t = 0) and after 30 days (t = 30 d). The release of the fragrance was measured at 36.5 °C (body temperature) by SPME-GC-MS.

### Examples

### 1. Measurement methods

In the following, measurement methods implemented in the examples are described.

### Particle size distribution

Volume determined median particle size *d*₅₀(vol) and the volume determined top cut particle size *d*₉₈(vol) was evaluated using a Malvern Mastersizer 3000 Laser Diffraction System (Malvern Instruments Plc., Great Britain). The *d*₅₀(vol) or *d*₉₈(vol) value indicates a diameter value such that 50 % or 98 % by volume, respectively, of the particles have a diameter of less than this value. The raw data obtained by the measurement was analyzed using the Mie theory, with a particle refractive index of 1.57 and an absorption index of 0.005. The methods and instruments are known to the skilled person and are commonly used to determine particle size distributions of fillers and pigments. The measurement was carried out in an aqueous solution of 0.1 wt.-% Na₄P₂O₇. The samples were dispersed using a high-speed stirrer and supersonicated.

### Specific surface area (SSA)

The specific surface area was measured via the BET method according to ISO 9277:2010 using nitrogen, following conditioning of the sample by heating at 250°C for a period of 30 minutes. Prior to such measurements, the sample was filtered within a Büchner funnel, rinsed with deionised water and dried at 110°C in an oven for at least 12 hours.

### Intra-particle intruded specific pore volume (in cm³/g)

The specific pore volume was measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 µm (~ nm). The equilibration time used at each pressure step was 20 seconds. The sample material was sealed in a 5 cm³ chamber powder penetrometer for analysis. The data were corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

The total pore volume seen in the cumulative intrusion data can be separated into two regions with the intrusion data from 214 µm down to about 1 - 4 µm showing the coarse packing of the sample between any agglomerate structures contributing strongly. Below these diameters lies the fine inter-particle packing of the particles themselves. If they also have intra-particle pores, then this region appears bi-modal, and by taking the specific pore volume intruded by mercury into pores finer than the modal turning point, i.e. finer than the bi-modal point of inflection, the specific intra-particle pore volume is defined. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the inter-particle pore region and the intra-particle pore region, if present. Knowing the intra-particle pore diameter range it is possible to subtract the remainder inter-particle and inter-agglomerate pore volume from the total pore volume to deliver the desired pore volume of the internal pores alone in terms of the pore volume per unit mass (specific pore volume). The same principle of subtraction, of course, applies for isolating any of the other pore size regions of interest.

### Sorption Kinetics

4 grams of material was taken in duplicates and the material were exposed for 360 min to the specific temperature and Relative Humidity (RH). The temperature had been kept constant for a complete cycle at 23°C and RH followed the given cycle, i.e. 10% - 30% - 50% - 70% - 85% - 70% - 50% - 30% - 10%. The weight change had been recorded at end of the cycle (360 minutes) and a graph had been plotted between change in weight at specific RH to reflect the sorption kinetic of the sample.

### Sensorial Analysis

The products were subjected to descriptive analysis (ref. Norm ISO 13299) carried out by a panel of eight assessors. The sessions were performed on 22nd May 2019 at 12.00 o'clock, at 14.30 o'clock and at 16.30 o'clock in a room dedicated to sensory analysis and equipped in compliance with UNI ISO 8589 standard. During a preliminary session, the Panel members, examining the products, developed a common vocabulary and contributed, together with the Panel leader, to define scales and terms of evaluation.

The following attributes were thus identified and defined:

### Visual attributes (appearance):

- Colour (product's colour as it is): light - dark
- Powder compaction (act of compacting): low - high
- *Attributes related to the first contact (pick up)*:Cushion effect (when the product is between the thumb and forefinger, the movement is dampened): low - high
- Slipperiness (ease of moving the product between the thumb and forefinger): low - high

### Attributes evaluated during the application:

- Spreadability (ease of distribution the product over the skin): low - high
- Covering (degree product covers the imperfection of the skin): low - high
- Whitening (degree skin remains white when the product is rubbed): low - high

### Attributes evaluated after the application (after-feel):

- Smoothness (degree skin, not marked by roughness): low - high
- Shiny on the skin (amount of light reflected on skin): low - high

The evaluation of each attribute was made using the same quantity of each sample. The product has been tested in a "blind" form and differentiated from the reference sample only with a code. Visual attributes were evaluated on the product as it is. For the attributes related to first contact, the assessment has been made between the fingertips and for those related to the product application and after-feel samples were tested on the defined area of the forearm. Using a skin scribe, 2 circles of 5,1 cm each were marked in the volar area of the forearm. The circle located near the elbows was identified as Site 1, and the circle located near the wrists was identified as Site 2. The detection sheet contained, for each attribute, non structured linear scales, defined at the extremities (0-10), where to indicate the placement of the sample under analysis. On the scales, the reference position has been assumed as central (value = 5) for all the sensory attributes because were considered to have potentially a higher or lower intensity. The assessor had to indicate on the scale the degree of intensity of the examined samples in comparison with the reference sample. Assessors made 3 evaluation sessions.

### Fragrance Release

The samples were analysed for their release properties stored absence of light and at T0 (immediately after loading) and after 30 days (T30). 1 g of the sample (with 3 replicates) was weighed into Head-Space Vials and intensively homogenized by the GC-MS Autosampler shaker. The release of the fragrance was measured at 36.5 °C (body temperature) by SPME-GC-MS.

### Patch Testing

The product is tested on 20 adult volunteers of both sexes, selected after the application of the criteria for inclusion / non-inclusion.

Excluded from the test:
- Children and persons below the age of consent
- Pregnant or lactating women
- Subjects affected by dermatitis
- Subjects with history of allergic skin reaction
- Subjects under anti-inflammatory drug therapy (either steroidal or non-steroidal)
- Subjects who participated in analogue tests in the last two months.

Before the test starts, all the participants are made aware of the purpose and nature of the study and of any foreseeable risks involved in participation in the study and give written informed consent to the experimentation. A form registering the date and the products tested is completed for each volunteer. The application of the product is made by technically qualified and trained persons with the supervision of a medical dermatologist. Skin evaluations are made by a dermatologist. It has been realized an occlusive patch test using the Curatest F patches applied to the volar area of the forearm.

The visual assessment of skin irritation is made in double: 24 hours after Curatest F application (30 minutes after patch removal) and 48 hours after Curatest F application (24 hours after patch removal). The evaluation parameters and the grading of skin reactions are reported here below:

### Erythema:

0 = no evidence of erythema; 0.5 = minimal or doubtful erythema; 1 = slight redness, spotty and diffuse; 2= moderate, uniform redness; 3=strong uniform redness; 4=fiery redness.

### Edema:

0 = no edema; 1 = light edema (hardly visible); 2 = light edema (clearly visible); 3 = moderate edema; 4 = strong edema (extended beyond the application area).

The sum of erythema and edema score is defined "irritation index". Irritation index value at 30 minutes and 24 hours after patch removal (24 and 48 hours after product application) are recorded on the volunteer's form.

### 2. The components

### Natural ground calcium carbonate NGCC

A high purity natural calcium carbonate having a *d*₅₀ (vol) in the range of 1.8 to 2.6 µm that is commercially available from Omya.

### Surface-reacted calcium carbonate SRCC 1

Surface-reacted calcium carbonate (SRCC 1) (*d*₅₀ (vol) = 6.6 µm, *d*₉₈ = 13.7 µm, SSA = 59.9 m²/g). The intra-particle intruded specific pore volume is 0.939 cm³/g (for the pore diameter range of 0.004 to 0.51 µm).

SRCC was obtained by preparing 350 litres of an aqueous suspension of ground calcium carbonate in a mixing vessel by adjusting the solids content of a ground limestone calcium carbonate from Omya SAS, Orgon having a weight based median particle size *d*₅₀(wt) of 1.3 µm, as determined by sedimentation, such that a solids content of 10 wt.-%, based on the total weight of the aqueous suspension, is obtained. Whilst mixing the slurry at a speed of 6.2 m/s, 11.2 kg phosphoric acid was added in form of an aqueous solution containing 30 wt.-% phosphoric acid to said suspension over a period of 20 minutes at a temperature of 70°C. After the addition of the acid, the slurry was stirred for additional 5 minutes, before removing it from the vessel and drying using a jet-dryer.

### Surface-reacted calcium carbonate SRCC 2

Surface-reacted calcium carbonate (SRCC 2) (*d*₅₀(vol) = 5.1 µm, *d*₉₈(vol) = 9.2 µm, SSA = 96.1 m²/g with an intra-particle intruded specific pore volume of 1.588 cm³/g (for the pore diameter range of 0.004 to 0.4 µm).

In a mixing vessel, 10 liters of an aqueous suspension of ground limestone calcium carbonate was prepared by adjusting the solids of a ground limestone calcium carbonate having a particle size distribution of 90 wt.-% below 2 µm, based on the total weight of the ground calcium carbonate, such that a solids content of 15 wt.-%, based on the total weight of the aqueous suspension, is obtained. Whilst mixing the slurry, 2.8 kg phosphoric acid was added in form of an aqueous solution containing 30 wt.-% phosphoric acid to said suspension over a period of 10 minutes. Throughout the whole experiment the temperature of the suspension was maintained at 70°C. After the addition of the acid, the suspension was stirred for additional 5 minutes before removing it from the vessel and drying.

### 3. Preparation of the dry cosmetic and/or skin care composition

Inventive composition 1 to 4 were prepared by the process according to section 3.a) or 3.b) below. Inventive composition 5 to 8 were prepared by the process according to section 3.a) or 3.b) as described below followed by the process according to section 3.c).

### a) Dry mixing of components

The calculated amount of the first component GCC1 in dry form and the second component SRCC1 or SRCC2 in dry form were put in an industrial spiral conical mixer and mixed. The spiral mixing blade inside the mixer initiates a three-dimensional flow of the mixing powders creating a helical upward movement on the periphery and downward flow in the center.

### b) Wet mixing of components

The first component GCC1 was provided in dry form and was re-suspended in an aqueous suspension of SRCC1 or SRCC2 in the indicated ratio. Then the suspended aqueous mixes were dried with the Riera Nadeu Jet Dryer and packed in bags independently.

### c) Loading of fragrance on the dry cosmetic and/or skin care composition

The dry cosmetic and/or skin care compositions were provided in powder form and were loaded in a Universal Tabletop Mixer Granulator (TMG 1/6, Glatt GmbH, Germany) with a rotor speed of 1000rpm and a chopper speed of 1000rpm. A 6L conical vessel was filled about 2/3 with one of the dry cosmetic and/or skin care compositions. The dry cosmetic and/or skin care composition was then premixed for three minutes, before the fragrance loading with a peristaltic pump (Modell 1B.1003-R/65, Petro Gas Ausrüstungen Berlin GmbH, Germany) was started. The loading was performed at a speed of 2.5 g/min until a fragrance content of 1.5% by weight was achieved. After the indicated amount of fragrance was reached, the peristaltic pump was switched of and the composition was mixed for one further minute.

### d) Examples

Inventive composition 1 (IC-1): NGCC 90 wt.%, SRCC1 10 wt.%.

Inventive composition 2 (IC-2): NGCC 90 wt.%, SRCC2 10 wt.%.

Inventive composition 3 (IC-3): NGCC 70 wt.%, SRCC1 30 wt.%.

Inventive composition 4 (IC-4): NGCC 70 wt.%, SRCC2 30 wt.%.

Inventive composition 5 (IC-5): First and second component 98.5 wt.% (NGCC:SRCC1; 9:1 by weight ratio), fragrance 1.5 wt.%.

Inventive composition 6 (IC-6): First and second component 98.5 wt.% (NGCC:SRCC2; 9:1 by weight ratio), fragrance 1.5 wt.%.

Inventive composition 7 (IC-7): First and second component 98.5 wt.% (NGCC:SRCC1; 7:3 by weight ratio), fragrance 1.5 wt.%.

Inventive composition 8 (IC-8): First and second component 98.5 wt.% (NGCC:SRCC2; 7:3 by weight ratio), fragrance 1.5 wt.%.

Comparative composition (CC-1): Talc 100 wt. %.

Comparative composition (CC-2): Talc 98.5 wt. %, fragrance 1.5 wt.%.

Comparative composition (CC-X): SRCC2 100 wt.%

Comparative composition (CC-Y): SRCC2 98.5 wt.%, fragrance 1.5 wt.%.

### 4. Results

### a) Sensorial analysis

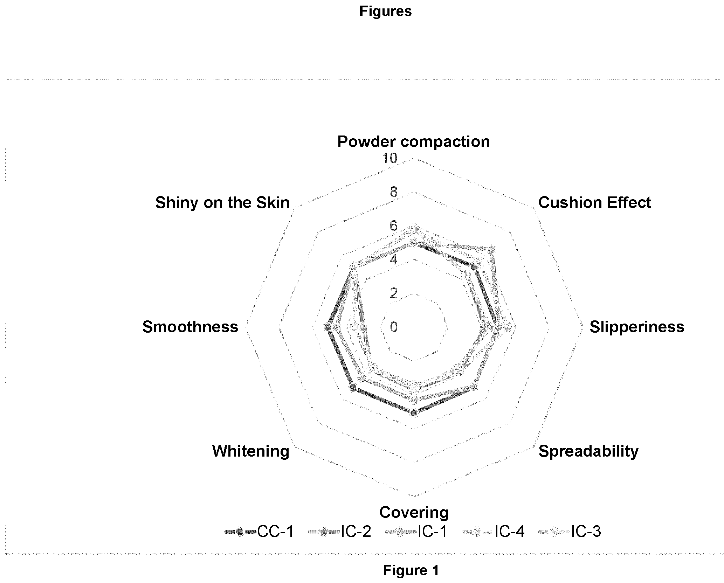

Figure 1 shows the results for the sensorial analysis of Inventive composition 1 to 4 and comparative composition 1. It can be gathered from Figure 1 that the inventive compositions show similar or better properties in almost every test aspect compared to the comparative composition 1. For example, all of the inventive compositions 1 to 4 show similar or better properties in terms of the cushion effect, the slipperiness and the powder compaction. Inventive composition 1 provided the best results.

Furthermore, Table 1 shows the results for the sensorial analysis of Inventive composition 1 to 4 in comparison with comparative composition CC-X (100% SRCC2).

**Table 1:**

| | **Composition** | | | | |
|---|---|---|---|---|---|
| **Sensorial property** | **CC-X** | **IC-1** | **IC-2** | **IC-3** | **IC-4** |
| Color | 7,1 | 8,1 | 7,53 | 7,98 | 7,8 |
| Powder compaction | 5 | 5 | 5,83 | 5,7 | 5,85 |
| Cushion Effect | 4,5 | 6,5 | 4,39 | 5,5 | 4,4 |
| Slipperiness | 5 | 5 | 4,2 | 5,52 | 4,51 |
| Spreadability | 1,8 | 5 | 3,8 | 3,8 | 3,55 |
| Covering | 1,8 | 4,3 | 3,69 | 3,45 | 3,6 |
| Whitening | 1,5 | 4,3 | 3,6 | 3,4 | 3,5 |
| Smoothness | 3,5 | 4,6 | 3 | 3,5 | 3,49 |
| Shiny on the Skin | 5 | 5 | 5 | 5,05 | 5 |

The data shows that the inventive compositions IC-1 to IC-4 show similar or better sensorial properties in all aspects than the comparative composition CC-X, which is a pure surface-reacted calcium carbonate. For example, the inventive compositions IC-1 to IC-4 show a better covering, whitening, and spreadability than the comparative composition CC-X.

### b) Patch Test

A patch test was performed as described above with each one of inventive composition 1 to 4 and with comparative composition 1.
b1) Patch test result for comparative composition 1 on sensitive skin:
   20 test subjects being of an age between 24 and 39 years and of male or female sex were tested. None of the test subjects showed evidence of erythema or edema after 30 minutes of patch removal (24 hours after application) or after 24 hours of patch removal (48 hours after application).
b2) Patch test result for the inventive composition 1 on sensitive skin:
   20 test subjects being of an age between 24 and 39 years and of male or female sex were tested. None of the test subjects showed evidence of erythema or edema after 30 minutes of patch removal (24 hours after application) or after 24 hours of patch removal (48 hours after application).
b3) Patch test result for the inventive composition 2 on sensitive skin:
   20 test subjects being of an age between 24 and 39 years and of male or female sex were tested. None of the test subjects showed evidence of erythema or edema after 30 minutes of patch removal (24 hours after application) or after 24 hours of patch removal (48 hours after application).
b4) Patch test result for the inventive composition 3 on sensitive skin:
   20 test subjects being of an age between 24 and 39 years and of male or female sex were tested. None of the test subjects showed evidence of erythema or edema after 30 minutes of patch removal (24 hours after application) or after 24 hours of patch removal (48 hours after application).
b5) Patch test result for the inventive composition 4 on sensitive skin:
   20 test subjects being of an age between 24 and 39 years and of male or female sex were tested. None of the test subjects showed evidence of erythema or edema after 30 minutes of patch removal (24 hours after application) or after 24 hours of patch removal (48 hours after application).

The patch test show that inventive compositions 1 to 4 are not irritant to sensitive skin.

### c) Sorption analysis

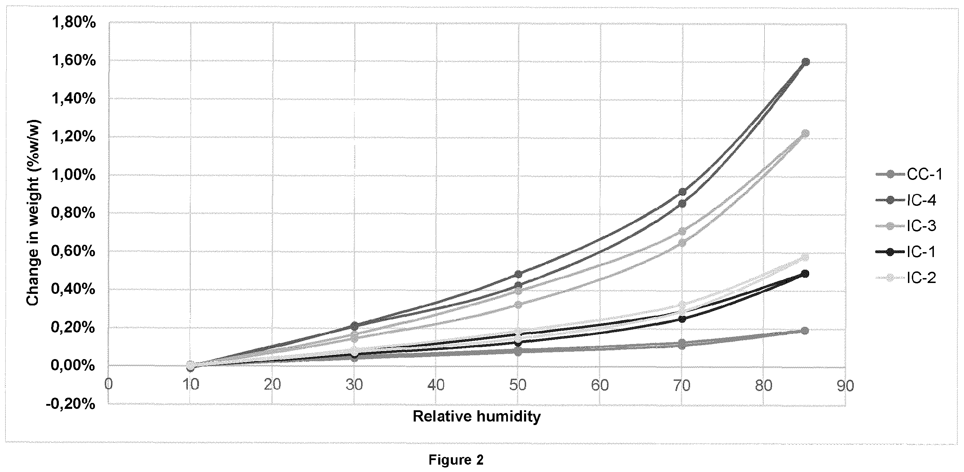

Figure 2 shows the results of the sorption analysis tests as described above carried out with inventive composition 1 to 4 and comparative composition 1. The comparative composition 1 comprising talc absorbed the least moisture or humidity from the air as shown by the least change in weight of all tested compositions. The inventive composition 3 and 4 comprising 30 wt.% of SRCC1 or SRCC2, respectively, showed the biggest change in weight of the composition due to moisture absorption. Inventive composition 1 and 2 comprising 10 wt.% of SRCC1 or SRCC2, respectively, absorbed less moisture than inventive composition 3 and 4, but more than the comparative composition 1. A high moisture absorption may be advantageous for a dry cosmetic and/or skin care composition, because it may allow for a better absorption of moisture on skin and/or body fluids.

### d) Fragrance release studies

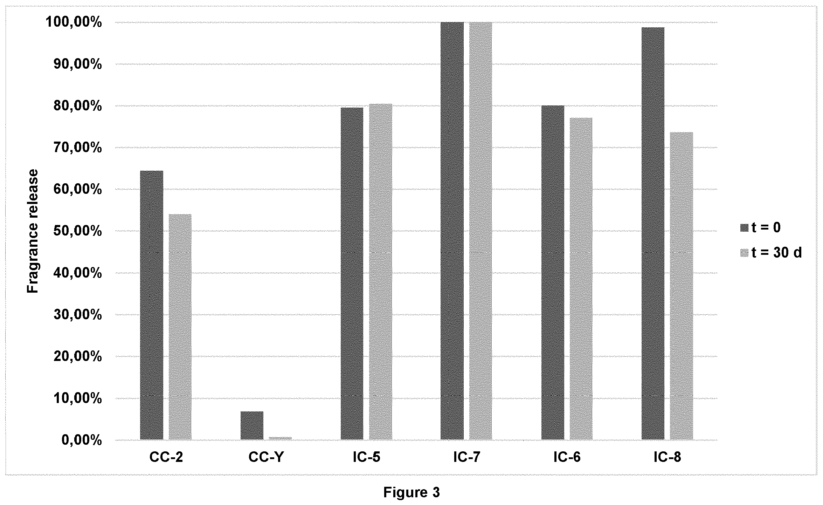

Figure 3 shows the results for fragrance release study tests as described above carried out with inventive composition 5 to 8 and comparative composition 2. Two different aspects can be compared by the data presented in Figure 3: (i) the percentage of fragrance released when the sample is subjected to 37°C, either directly after loading (t = 0) or after 30 days of storage (t = 30), and (b) the difference between fragrance release on time t = 0 and time t = 30.

Regarding aspect (i), it can be gathered from Figure 3 that each one of inventive composition 5 to 8 releases more fragrance from the composition on time t = 0, i.e. directly after loading, than the comparative composition 2. More precisely, the comparative composition releases approx. 65% of the loaded fragrance when subjected to a temperature of 37°C, whereas each one of the inventive compositions 5 to 8 releases at least approx. 80% of the fragrance. Inventive composition 7 and 8 release almost all of the loaded fragrance on time t = 0. After 30 days of storage, the comparative composition 2 releases only approx. 54% of the initially loaded fragrance compared to at least 73% of the fragrance for each one of the inventive compositions 5 to 8. Inventive composition 7 releases almost 100% of the initially loaded fragrance after 30 days of storage. Thus, the results show that the inventive compositions 5 to 8 show a better fragrance release compared to the comparative composition 2 on time t = 0, i.e. directly after loading, and on time t = 30, i.e. after 30 days of storage.

With respect to aspect (ii) of the results, it is to be noted that the difference in fragrance release for a specific composition measured on time t = 0 and time t = 30 is an indicator for the stability of the composition during storage. For example, a composition might release 100% fragrance immediately after loading, but only 50% fragrance after storage over 30 days. This would be an indicator for an instability or decomposition of the fragrance and/or the composition, since the release profile is different. Comparative composition 2 releases on time t = 0 approx. 65% of the fragrance and on time t = 30 approx. 54% of the fragrance, which corresponds to a difference of approx. 11%. Thus, it can be gathered from Fig. 3 that the comparative composition 2 shows a certain instability or decomposition during storage. In contrast thereto, inventive composition 5 to 7 show almost the same, if not the same, release profile on time t = 0 and on time t = 30. Thus, the inventive compositions 5 to 7 show no, or almost no, instability or decomposition during storage. It can be concluded therefrom that the inventive compositions 5 to 7 are more stable compared to comparative composition 2.

Furthermore, Table 2 as well as Figure 3 shows the results for the fragrance release test of Inventive compositions IC-5 to IC-8 in comparison with comparative composition CC-Y (98.5 wt.% SRCC2; 1.5 wt.% fragrance).

**Table 2:**

| | **Fragrance release** | |
|---|---|---|
| Time | t = 0 | t = 30 d |
| CC-Y | 6,80% | 0,70% |
| IC-5 | 79,50% | 80,40% |
| IC-6 | 80,00% | 77,10% |
| IC-7 | 100% | 100% |
| IC-8 | 98,70% | 73,60% |

The data in Table 2 and Figure 3 shows that the inventive compositions IC-5 to IC-8 have a much higher fragrance release directly after leading (t = 0) and after 30 days of storage (t = 30 d) than the comparative composition CC-Y, which is a surface-reacted calcium carbonate combined with fragrance. Moreover, the data in Table 2 shows that the drop of fragrance release during storage, i.e. from t = 0 to t = 30 d, is much higher for CC-Y than for IC-5 to IC-8, which means that the stability of the fragrance in CC-Y is very low. For IC-5 to IC-8 there is either no decrease in fragrance release over time, or only a minor decrease. Thus, the stability of IC-5 to IC-8 is much better than the stability of CC-Y.

## Claims

1. A dry cosmetic and/or skin care composition comprising a mixture of
a first component being a natural ground calcium carbonate, and
a second component being a surface-reacted calcium carbonate, wherein
the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
wherein the dry cosmetic and/or skin care composition contains a water content of less than 15 wt.%, based on the total weight of the cosmetic and/or skin care composition.

2. The dry cosmetic and/or skin care composition according to claim 1, wherein the dry cosmetic and/or skin care composition is a powder, preferably a baby powder and/or a body powder.

3. The dry cosmetic and/or skin care composition according to claim 1 or 2, wherein the first component is present in an amount of from 1 wt.% to 99 wt.%, preferably from 30 wt.% to 99 wt.%, more preferably from 50 wt.% to 95 wt.%, even more preferably from 60 wt.% to 95 wt.%, and most preferably from 70 wt.% to 90 wt.%, based on the total weight of the first component and the second component, and
wherein the second component is present in an amount of from 1 wt.% to 99 wt.%, preferably from 1 wt.% to 70 wt.%, more preferably from 5 wt.% to 50 wt.%, even more preferably from 5 wt.% to 40 wt.%, and most preferably from 10 wt.% to 30 wt.%, based on the total weight of the first component and the second component.

4. The dry cosmetic and/or skin care composition according to any one of the preceding claims, wherein the first component is a natural ground calcium carbonate selected from the group consisting of marble, chalk, limestone, and mixtures thereof.

5. The dry cosmetic and/or skin care composition according to any one of the preceding claims, wherein the surface-reacted calcium carbonate is a reaction product of natural ground calcium carbonate selected from the group consisting of marble, chalk, limestone, and mixtures thereof, with carbon dioxide and at least one H₃O⁺ ion donor, wherein the carbon dioxide is formed in situ by the at least one H₃O⁺ ion donor treatment and/or is supplied from an external source, or
wherein the surface-reacted calcium carbonate is a reaction product of precipitated calcium carbonate selected from the group consisting of precipitated calcium carbonates having an aragonitic, vateritic or calcitic crystal form, and mixtures thereof, with carbon dioxide and at least one H₃O⁺ ion donor, wherein the carbon dioxide is formed in situ by the at least one H₃O⁺ ion donor treatment and/or is supplied from an external source.

6. The dry cosmetic and/or skin care composition according to any one of the preceding claims, wherein the at least one H₃O⁺ ion donor is selected from the group consisting of hydrochloric acid, sulphuric acid, sulphurous acid, phosphoric acid, citric acid, oxalic acid, acetic acid, formic acid, and mixtures thereof, preferably the at least one H₃O⁺ ion donor is selected from the group consisting of hydrochloric acid, sulphuric acid, sulphurous acid, phosphoric acid, oxalic acid, H₂PO₄⁻, being at least partially neutralised by a cation selected from Li⁺, Na⁺ and/or K⁺, HPO₄²⁻, being at least partially neutralised by a cation selected from Li⁺, Na^{+,} K⁺, Mg²⁺, and/or Ca²⁺, and mixtures thereof, more preferably the at least one H₃O⁺ ion donor is selected from the group consisting of hydrochloric acid, sulphuric acid, sulphurous acid, phosphoric acid, oxalic acid, or mixtures thereof, and most preferably, the at least one H₃O⁺ ion donor is phosphoric acid.

7. The dry cosmetic and/or skin care composition according to any one of the preceding claims, wherein the first component has a volume median particle size *d*₅₀ from 0.1 to 50 µm, preferably from 0.5 to 40 µm, more preferably from 0.5 to 20 µm, even more preferably from 0.5 to 10 µm, and most preferably from 0.8 to 8 µm, and/or
wherein the first component has a specific surface area of from 0.5 m²/g to 30 m²/g, preferably from 1 m²/g to 20 m²/g, and more preferably from 2 m²/g to 15 m²/g, measured using nitrogen and the BET method.

8. The dry cosmetic and/or skin care composition according to any one of the preceding claims, wherein the second component has a volume median particle size *d*₅₀ from 0.5 to 50 µm, preferably from 1 to 40 µm, more preferably from 1.2 to 30 µm, and even more preferably from 1.5 to 15 µm, and most preferably from 3 to 10 µm, and/or
wherein the second component has a specific surface area of from 15 m²/g to 200 m²/g, preferably from 20 m²/g to 180 m²/g, more preferably from 25 m²/g to 160 m²/g, and most preferably from 30 m²/g to 90 m²/g, measured using nitrogen and the BET method.

9. The dry cosmetic and/or skin care composition according to any one of the preceding claims, wherein the dry cosmetic and/or skin care composition is free of talc or a talc-containing material.

10. The dry cosmetic and/or skin care composition according to any one of the preceding claims, wherein the dry cosmetic and/or skin care composition comprises one or more of a further component, preferably the one or more further component is selected from the group consisting of a fragrance, an aroma, an antibacterial and/or an antiseptic agent, a fatty acid or a salt thereof, a fatty alcohol, a vegetable or a synthetic oil, a polymeric carbohydrate, a mineral additive, a pigment, a salt, and mixtures thereof.

11. A process for preparing a dry cosmetic and/or skin care composition according to any one of claims 1 to 10, wherein the process comprises the following steps:
a) providing a first component being a natural ground calcium carbonate,
b) providing a second component being a surface-reacted calcium carbonate, wherein
the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
c) mixing the first component of step a) with the second component of step b).

12. The process according to claim 11, wherein the first component of step a) and the second component of step b) is provided in dry form and wherein mixing step c) is a dry blending step, wherein the first component of step a) has a water content of below 3.0 wt.%, based on the total dry weight of the first component, and the second component of step b) has a water content of below 10.0 wt.%, based on the total dry weight of the second component, or
wherein the first component of step a) and/or the second component of step b) is provided in form of an aqueous suspension, preferably in form of a slurry, and wherein the process further comprises a step d) of drying the mixture obtained in step c), preferably step d) is a spray drying step or a superheated steam drying step, and more preferably a spray drying step.

13. Use of a dry composition comprising a mixture of
a first component being a natural ground calcium carbonate, and
a second component being a surface-reacted calcium carbonate, wherein
the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
as a cosmetic and/or skin care composition, preferably as a cosmetic and/or skin care powder, and most preferably as a baby powder and/or a body powder.

14. The use according to claim 13, wherein the dry composition is used for absorbing fluids, for decreasing skin friction, for modifying the skin feel, and/or for modifying the skin appearance.

15. Use of a mixture comprising
a first component being a natural ground calcium carbonate, and
a second component being a surface-reacted calcium carbonate, wherein
the surface-reacted calcium carbonate is a reaction product of natural ground calcium carbonate or precipitated calcium carbonate with carbon dioxide and at least one H₃O⁺ ion donor, wherein the carbon dioxide is formed in situ by the at least one H₃O⁺ ion donor treatment and/or is supplied from an external source,
as a replacement for talc or a talc-containing material in a dry cosmetic and/or skin care composition.

## Patentansprüche

1. Trockene Kosmetik- und/oder Hautpflegezusammensetzung, umfassend ein Gemisch aus
einer ersten Komponente, die ein natürliches gemahlenes Calciumcarbonat ist, und
einer zweiten Komponente, die ein oberflächenreagiertes Calciumcarbonat ist, wobei
das oberflächenreagierte Calciumcarbonat ein Reaktionsprodukt von natürlichem gemahlenem Calciumcarbonat oder ausgefälltem Calciumcarbonat mit Kohlendioxid und zumindest einem H₃O⁺-lonendonator ist, wobei das Kohlendioxid in situ durch die Behandlung mit dem zumindest einen H₃O⁺-Ionendonator gebildet und/oder aus einer externen Quelle zugeführt wird,
wobei die trockene Kosmetik- und/oder Hautpflegezusammensetzung einen Wassergehalt von weniger als 15 Gew.-%, bezogen auf das Gesamtgewicht der Kosmetik- und/oder Hautpflegezusammensetzung, enthält.

2. Trockene Kosmetik- und/oder Hautpflegezusammensetzung nach Anspruch 1, wobei die trockene Kosmetik- und/oder Hautpflegezusammensetzung ein Puder, bevorzugt ein Babypuder und/oder ein Körperpuder ist.

3. Trockene Kosmetik- und/oder Hautpflegezusammensetzung nach Anspruch 1 oder 2, wobei die erste Komponente in einer Menge von 1 Gew.-% bis 99 Gew.-%, bevorzugt von 30 Gew.-% bis 99 Gew.-%, bevorzugter von 50 Gew.-% bis 95 Gew.-%, noch bevorzugter von 60 Gew.-% bis 95 Gew.-% und besonders bevorzugt von 70 Gew.-% bis 90 Gew.-%, bezogen auf das Gesamtgewicht der ersten Komponente und der zweiten Komponente, vorhanden ist, und
wobei die zweite Komponente in einer Menge von 1 Gew.-% bis 99 Gew.-%, bevorzugt von 1 Gew.-% bis 70 Gew.-%, bevorzugter von 5 Gew.-% bis 50 Gew.-%, noch bevorzugter von 5 Gew.-% bis 40 Gew.-% und besonders bevorzugt von 10 Gew.-% bis 30 Gew.-%, bezogen auf das Gesamtgewicht der ersten Komponente und der zweiten Komponente, vorhanden ist.

4. Trockene Kosmetik- und/oder Hautpflegezusammensetzung nach einem der vorstehenden Ansprüche, wobei die erste Komponente ein natürliches gemahlenes Calciumcarbonat, ausgewählt aus der Gruppe bestehend aus Marmor, Kalk, Kalkstein und Gemischen davon, ist.

5. Trockene Kosmetik- und/oder Hautpflegezusammensetzung nach einem der vorstehenden Ansprüche, wobei das oberflächen reagierte Calciumcarbonat ein Reaktionsprodukt von natürlichem gemahlenem Calciumcarbonat, ausgewählt aus der Gruppe bestehend aus Marmor, Kalk, Kalkstein und Gemischen davon, mit Kohlendioxid und zumindest einem H₃O'-Ionendonator ist, wobei das Kohlendioxid in situ durch die Behandlung mit dem zumindest einem H₃O⁺-Ionendonator gebildet und/oder aus einer externen Quelle zugeführt wird, oder
wobei das oberflächenreagierte Calciumcarbonat ein Reaktionsprodukt von ausgefälltem Calciumcarbonat, ausgewählt aus der Gruppe bestehend aus ausgefällten Calciumcarbonaten mit einer aragonitischen, vateritischen oder calcitischen Kristallform und Gemischen davon, mit Kohlendioxid und zumindest einem H₃O⁺-Ionendonator ist, wobei das Kohlendioxid in situ durch die Behandlung mit dem zumindest einem H₃O⁺-Ionendonator gebildet und/oder aus einer externen Quelle zugeführt wird.

6. Trockene Kosmetik- und/oder Hautpflegezusammensetzung nach einem der vorstehenden Ansprüche, wobei der zumindest eine H₃O⁺-Ionendonator ausgewählt ist aus der Gruppe bestehend aus Salzsäure, Schwefelsäure, schwefliger Säure, Phosphorsäure, Citronensäure, Oxalsäure, Essigsäure, Ameisensäure und Gemischen davon, wobei der zumindest eine H₃O⁺-Ionendonator bevorzugt ausgewählt ist aus der Gruppe bestehend aus Salzsäure, Schwefelsäure, schwefliger Säure, Phosphorsäure, Oxalsäure, H₂PO₄⁻, zumindest teilweise neutralisiert durch ein Kation, ausgewählt aus Li⁺, Na⁺ und/oder K⁺, HPO₄², zumindest teilweise neutralisiert durch ein Kation ausgewählt aus Li⁺, Na⁺, K⁺, Mg²⁺ und/oder Ca²⁺ und Gemischen davon, bevorzugter wobei der zumindest eine H₃O⁺-Ionendonator aus der Gruppe ausgewählt ist, bestehend aus Salzsäure, Schwefelsäure, schwefliger Säure, Phosphorsäure, Oxalsäure oder Gemischen davon, und besonders bevorzugt wobei der zumindest eine H₃O⁺-Ionendonator Phosphorsäure ist.

7. Trockene Kosmetik- und/oder Hautpflegezusammensetzung nach einem der vorstehenden Ansprüche, wobei die erste Komponente eine volumengemittelte Partikelgröße *d*₅₀ von 0,1 bis 50 µm, bevorzugt von 0,5 bis 40 µm, bevorzugter von 0,5 bis 20 µm, noch bevorzugter von 0,5 bis 10 µm und besonders bevorzugt von 0,8 bis 8 µm aufweist, und/oder
wobei die erste Komponente eine spezifische Oberfläche von 0,5 m²/g bis 30 m²/g, bevorzugt von 1 m²/g bis 20 m²/g, und bevorzugter von 2 m²/g bis 15 m²/g aufweist, gemessen unter Verwendung von Stickstoff und der BET-Methode.

8. Trockene Kosmetik- und/oder Hautpflegezusammensetzung nach einem der vorstehenden Ansprüche, wobei die zweite Komponente eine volumengemittelte Partikelgröße *d*₅₀ von 0,5 bis 50 µm, bevorzugt von 1 bis 40 µm, bevorzugter von 1,2 bis 30 µm, noch bevorzugter von 1,5 bis 15 µm und besonders bevorzugt von 3 bis 10 µm aufweist, und/oder
wobei die zweite Komponente eine spezifische Oberfläche von 15 m²/g bis 200 m²/g, bevorzugt von 20 m²/g bis 180 m²/g, bevorzugter von 25 m²/g bis 160 m²/g, und besonders bevorzugt von 30 m²/g bis 90 m²/g aufweist, gemessen unter Verwendung von Stickstoff und der BET-Methode.

9. Trockene Kosmetik- und/oder Hautpflegezusammensetzung nach einem der vorstehenden Ansprüche, wobei die trockene Kosmetik- und/oder Hautpflegezusammensetzung frei von Talk oder talkhaltigen Materialien ist.

10. Trockene Kosmetik- und/oder Hautpflegezusammensetzung nach einem der vorstehenden Ansprüche, wobei die trockene Kosmetik- und/oder Hautpflegezusammensetzung eine oder mehrere weitere Komponenten umfasst, wobei bevorzugt die eine oder mehreren weiteren Komponenten ausgewählt sind aus der Gruppe bestehend aus einem Duftstoff, einem Aroma, einem antibakteriellen und/oder einem antiseptischem Mittel, einer Fettsäure oder einem Salz davon, einem Fettalkohol, einem pflanzlichen oder synthetischen Öl, einem polymeren Kohlenhydrat, einem mineralischen Zusatzstoff, einem Pigment, einem Salz und Gemischen davon.

11. Prozess zum Herstellen einer trockenen Kosmetik- und/oder Hautpflegezusammensetzung nach einem der Ansprüche 1 bis 10, wobei der Prozess die folgenden Schritte umfasst:
a) Bereitstellen einer ersten Komponente, die ein natürliches gemahlenes Calciumcarbonat ist,
b) Bereitstellen einer zweiten Komponente, die ein oberflächenreagiertes Calciumcarbonat ist, wobei
das oberflächenreagierte Calciumcarbonat ein Reaktionsprodukt von natürlichem gemahlenem Calciumcarbonat oder ausgefälltem Calciumcarbonat mit Kohlendioxid und zumindest einem H₃O⁺-lonendonator ist, wobei das Kohlendioxid in situ durch die Behandlung mit dem zumindest einen H₃O⁺-Ionendonator gebildet und/oder aus einer externen Quelle zugeführt wird,
c) Mischen der ersten Komponente aus Schritt a) mit der zweiten Komponente aus Schritt b).

12. Prozess nach Anspruch 11, wobei die erste Komponente aus Schritt a) und die zweite Komponente aus Schritt b) in trockener Form bereitgestellt werden und wobei Mischschritt c) ein Trockenmischschritt ist, wobei die erste Komponente aus Schritt a) einen Wassergehalt von unter 3,0 Gew.-% aufweist, bezogen auf das Gesamttrockengewicht der ersten Komponente, und die zweite Komponente aus Schritt b) einen Wassergehalt von unter 10,0 Gew.-%, bezogen auf das Gesamttrockengewicht der zweiten Komponente, aufweist, oder
wobei die erste Komponente aus Schritt a) und/oder die zweite Komponente aus Schritt b) in Form einer wässrigen Suspension, bevorzugt in Form einer Aufschlämmung, bereitgestellt werden, und wobei der Prozess weiter einen Schritt d) zum Trocknen des in Schritt c) erhaltenen Gemisches umfasst, wobei Schritt d) bevorzugt ein Sprühtrocknungsschritt oder ein Trocknungsschritt mit überhitztem Dampf und bevorzugter ein Sprühtrocknungsschritt ist.

13. Verwendung einer trockenen Zusammensetzung, umfassend ein Gemisch aus
einer ersten Komponente, die ein natürliches gemahlenes Calciumcarbonat ist, und
einer zweiten Komponente, die ein oberflächenreagiertes Calciumcarbonat ist, wobei
das oberflächenreagierte Calciumcarbonat ein Reaktionsprodukt von natürlichem gemahlenem Calciumcarbonat oder ausgefälltem Calciumcarbonat mit Kohlendioxid und zumindest einem H₃O⁺-lonendonator ist, wobei das Kohlendioxid in situ durch die Behandlung mit dem zumindest einen H₃O⁺-Ionendonator gebildet und/oder aus einer externen Quelle zugeführt wird,
als eine Kosmetik- und/oder Hautpflegezusammensetzung, bevorzugt als ein Kosmetik- und/oder Hautpflegepuder und besonders bevorzugt als ein Babypuder und/oder ein Körperpuder.

14. Verwendung nach Anspruch 13, wobei die trockene Zusammensetzung zum Aufnehmen von Flüssigkeiten, zum Verringern von Hautreibung, zum Verändern des Hautgefühls und/oder zum Verändern des Hautbildes verwendet wird.

15. Verwendung eines Gemisches aus
einer ersten Komponente, die ein natürliches gemahlenes Calciumcarbonat ist, und
einer zweiten Komponente, die ein oberflächenreagiertes Calciumcarbonat ist, wobei
das oberflächenreagierte Calciumcarbonat ein Reaktionsprodukt von natürlichem gemahlenem Calciumcarbonat oder ausgefälltem Calciumcarbonat mit Kohlendioxid und zumindest einem H₃O⁺-lonendonator ist, wobei das Kohlendioxid in situ durch die Behandlung mit dem zumindest einen H₃O⁺-Ionendonator gebildet und/oder aus einer externen Quelle zugeführt wird,
als Ersatz für Talk oder talkhaltige Stoffe in einer trockenen Kosmetik- und/oder Hautpflegezusammensetzung.

## Revendications

1. Composition sèche cosmétique et/ou de soins de la peau comprenant un mélange de
un premier composant étant un carbonate de calcium naturel broyé, et
un second composant étant un carbonate de calcium ayant réagi en surface, dans laquelle
le carbonate de calcium ayant réagi en surface est un produit de réaction du carbonate de calcium naturel broyé ou du carbonate de calcium précipité avec du dioxyde de carbone et au moins un donneur d'ions H₃O⁺, dans laquelle le dioxyde de carbone est formé in situ par traitement du au moins un donneur d'ions H₃O⁺ et/ou est fourni par une source externe,
dans laquelle la composition sèche cosmétique et/ou de soins de la peau contient une teneur en eau inférieure à 15 % en poids, sur la base du poids total de la composition cosmétique et/ou de soins de la peau.

2. Composition sèche cosmétique et/ou de soins de la peau selon la revendication 1, dans laquelle la composition sèche cosmétique et/ou de soins de la peau est une poudre, de préférence une poudre pour bébé et/ou une poudre pour le corps.

3. Composition sèche cosmétique et/ou de soins de la peau selon la revendication 1 ou 2, dans laquelle le premier composant est présent en une quantité allant de 1 % en poids à 99 % en poids, de préférence de 30 % en poids à 99 % en poids, plus préférentiellement de 50 % en poids à 95 % en poids, encore plus préférentiellement de 60 % en poids à 95 % en poids, et le plus préférentiellement de 70 % en poids à 90 % en poids, sur la base du poids total du premier composant et du second composant, et
dans laquelle le second composant est présent en une quantité allant de 1 % en poids à 99 % en poids, de préférence de 1 % en poids à 70 % en poids, plus préférentiellement de 5 % en poids à 50 % en poids, encore plus préférentiellement de 5 % en poids à 40 % en poids, et le plus préférentiellement de 10 % en poids à 30 % en poids, sur la base du poids total du premier composant et du second composant.

4. Composition sèche cosmétique et/ou de soins de la peau selon l'une quelconque des revendications précédentes, dans laquelle le premier composant est un carbonate de calcium naturel broyé sélectionné dans le groupe constitué par le marbre, la craie, le calcaire et des mélanges de ceux-ci.

5. Composition sèche cosmétique et/ou de soins de la peau selon l'une quelconque des revendications précédentes, dans laquelle le carbonate de calcium ayant réagi en surface est un produit de réaction de carbonate de calcium naturel broyé sélectionné dans le groupe constitué par le marbre, la craie, le calcaire et des mélanges de ceux-ci, avec du dioxyde de carbone et au moins un donneur d'ions H₃O⁺, dans laquelle le dioxyde de carbone est formé in situ par traitement du au moins un donneur d'ions H₃O⁺ et/ou est fourni à partir d'une source externe, ou
dans laquelle le carbonate de calcium ayant réagi en surface est un produit de réaction de carbonate de calcium précipité sélectionné dans le groupe constitué par des carbonates de calcium précipités présentant une forme cristalline aragonitique, vatéritique ou calcitique et des mélanges de celles-ci, avec du dioxyde de carbone et au moins un donneur d'ions H₃O⁺, dans laquelle le dioxyde de carbone est formé in situ par traitement du au moins un donneur d'ions H₃O⁺ et/ou est fourni à partir d'une source externe.

6. Composition sèche cosmétique et/ou de soins de la peau selon l'une quelconque des revendications précédentes, dans laquelle le au moins un donneur d'ions H₃O⁺ est sélectionné dans le groupe constitué par l'acide chlorhydrique, l'acide sulfurique, l'acide sulfureux, l'acide phosphorique, l'acide citrique, l'acide oxalique, l'acide acétique, l'acide formique et des mélanges de ceux-ci, de préférence le au moins un donneur d'ions H₃O⁺ est sélectionné dans le groupe constitué par l'acide chlorhydrique, l'acide sulfurique, l'acide sulfureux, l'acide phosphorique, l'acide oxalique, H₂PO₄⁻, étant au moins partiellement neutralisé par un cation sélectionné parmi Li⁺, Na⁺ et/ou K⁺, HPO₄²⁻, étant au moins partiellement neutralisé par un cation sélectionné parmi Li⁺, Na⁺, K⁺, Mg²⁺ et/ou Ca²⁺, et des mélanges de ceux-ci, plus préférentiellement le au moins un donneur d'ions H₃O⁺ est sélectionné dans le groupe constitué par l'acide chlorhydrique, l'acide sulfurique, l'acide sulfureux, l'acide phosphorique, l'acide oxalique, ou des mélanges de ceux-ci, et le plus préférentiellement le au moins un donneur d'ions H₃O⁺ est l'acide phosphorique.

7. Composition sèche cosmétique et/ou de soins de la peau selon l'une quelconque des revendications précédentes, dans laquelle le premier composant présente une taille de particule moyenne en volume *d*₅₀ de 0,1 à 50 µm, de préférence de 0,5 à 40 µm, plus préférentiellement de 0,5 à 20 µm, encore plus préférentiellement de 0,5 à 10 µm, et le plus préférentiellement de 0,8 à 8 µm, et/ou
dans laquelle le premier composant présente une surface spécifique de 0,5 m²/g à 30 m²/g, de préférence de 1 m²/g à 20 m²/g, et plus préférentiellement de 2 m²/g à 15 m²/g, mesurée à l'aide d'azote et du procédé BET.

8. Composition sèche cosmétique et/ou de soins de la peau selon l'une quelconque des revendications précédentes, dans laquelle le second composant présente une taille de particule moyenne en volume *d*₅₀ de 0,5 à 50 µm, de préférence de 1 à 40 µm, plus préférentiellement de 1,2 à 30 µm, et encore plus préférentiellement de 1,5 à 15 µm, et le plus préférentiellement de 3 à 10 µm, et/ou
dans laquelle le second composant présente une surface spécifique de 15 m²/g à 200 m²/g, de préférence de 20 m²/g à 180 m²/g, plus préférentiellement de 25 m²/g à 160 m²/g, et le plus préférentiellement de 30 m²/g à 90 m²/g, mesurée à l'aide d'azote et de la méthode BET.

9. Composition sèche cosmétique et/ou de soins de la peau selon l'une quelconque des revendications précédentes, dans laquelle la composition sèche cosmétique et/ou de soins de la peau est exempte de talc ou de matière contenant du talc.

10. Composition sèche cosmétique et/ou de soins de la peau selon l'une quelconque des revendications précédentes, dans laquelle la composition sèche cosmétique et/ou de soins de la peau comprend un ou plusieurs composants supplémentaires, de préférence les un ou plusieurs composants supplémentaires sont sélectionnés dans le groupe constitué d'un parfum, un arôme, un agent antibactérien et/ou antiseptique, un acide gras ou un sel de celui-ci, un alcool gras, une huile végétale ou synthétique, un glucide polymère, un additif minéral, un pigment, un sel et des mélanges de ceux-ci.

11. Processus de préparation d'une composition sèche cosmétique et/ou de soins de la peau selon l'une quelconque des revendications 1 à 10, dans lequel le processus comprend les étapes de :
a) fourniture d'un premier composant étant un carbonate de calcium naturel broyé,
b) fourniture d'un second composant étant un carbonate de calcium ayant réagi en surface, dans lequel
le carbonate de calcium ayant réagi en surface est un produit de réaction du carbonate de calcium naturel broyé ou du carbonate de calcium précipité avec du dioxyde de carbone et au moins un donneur d'ions H₃O⁺, dans laquelle le dioxyde de carbone est formé in situ par traitement du au moins un donneur d'ions H₃O⁺ et/ou est fourni par une source externe,
c) mélange du premier composant de l'étape a) avec le second composant de l'étape b).

12. Processus selon la revendication 11, dans lequel le premier composant de l'étape a) et le second composant de l'étape b) sont fournis sous forme sèche et dans lequel l'étape c) de mélange est une étape de mélange à sec, dans lequel le premier composant de l'étape a) présente une teneur en eau inférieure à 3,0 % en poids, sur la base du poids sec total du premier composant, et le second composant de l'étape b) présente une teneur en eau inférieure à 10,0 % en poids, sur la base du poids sec total du second composant, ou
dans lequel le premier composant de l'étape a) et/ou le second composant de l'étape b) est fourni sous forme d'une suspension aqueuse, de préférence sous forme d'une bouillie, et dans lequel le processus comprend en outre une étape d) de séchage du mélange obtenu à l'étape c), de préférence l'étape d) est une étape de séchage par pulvérisation ou une étape de séchage à la vapeur surchauffée, et plus préférentiellement une étape de séchage par pulvérisation.

13. Utilisation d'une composition sèche comprenant un mélange de
un premier composant étant un carbonate de calcium naturel broyé, et
un second composant étant un carbonate de calcium ayant réagi en surface, dans laquelle
le carbonate de calcium ayant réagi en surface est un produit de réaction du carbonate de calcium naturel broyé ou du carbonate de calcium précipité avec du dioxyde de carbone et au moins un donneur d'ions H₃O⁺, dans laquelle le dioxyde de carbone est formé in situ par traitement du au moins un donneur d'ions H₃O⁺ et/ou est fourni par une source externe,
comme composition cosmétique et/ou de soins de la peau, de préférence comme poudre cosmétique et/ou de soins de la peau, et le plus préférentiellement comme poudre pour bébé et/ou poudre pour le corps.

14. Utilisation selon la revendication 13, dans laquelle la composition sèche est utilisée pour absorber des fluides, pour diminuer le frottement cutané, pour modifier la sensation cutanée, et/ou pour modifier l'apparence de la peau.

15. Utilisation d'un mélange comprenant
fourniture d'un premier composant étant un carbonate de calcium naturel broyé,
un second composant étant un carbonate de calcium ayant réagi en surface, dans laquelle
le carbonate de calcium ayant réagi en surface est un produit de réaction du carbonate de calcium naturel broyé ou du carbonate de calcium précipité avec du dioxyde de carbone et au moins un donneur d'ions H₃O⁺, dans laquelle le dioxyde de carbone est formé in situ par traitement du au moins un donneur d'ions H₃O⁺ et/ou est fourni par une source externe,
en remplacement du talc ou d'une matière contenant du talc dans une composition sèche cosmétique et/ou de soins de la peau.
